# EUROPEAN PATENT SPECIFICATION

(11) **EP 1 742 957 B1**
(45) Date of publication and mention of the grant of the patent: **12.09.2007**
(21) Application number: 05775551.4
(22) Date of filing: 25.04.2005
(51) Int. Cl.: C07H 17/08

(54) **PROCESS FOR THE PREPARATION OF TELITHROMYCIN**
VERFAHREN ZUR VORBEREITUNG VON TELITHROMYCIN
PROCEDE DE PREPARATION DE LA TELITHROMYCINE

(30) Priority: 28.04.2004 IN MU04912004
(43) Date of publication of application: 17.01.2007
(73) Proprietor: Alembic Limited, Gujarat, Vadodara 390 003 (IN)
(72) Inventor: SOHANI, Suhas, Gujarat, Vadodara 390 003 (IN); DEODHAR, Mandar, Gujarat, Vadodara 390 003 (IN); PATEL, Nishant, Gujarat, Vadodara 390 003 (IN); PATEL, Manish, Gujarat, Vadodara 390 003 (IN); DAVADRA, Mahesh, Gujarat, Vadodara 390 003 (IN); KANSAL, Vinodkumar, Gujarat, Vadodara 390 003 (IN)
(74) Representative: Harrison Goddard Foote
(86) International application number: PCT/IN2005/000125
(87) International publication number: WO 2005/105821

(56) References cited:
- EP-A- 0 487 411
- FR-A1- 2 732 684
- US-A- 5 635 485
- WU, YONG-JIN ET AL: "Recent developments on ketolides and macrolides" CURRENT MEDICINAL CHEMISTRY , 8(14), 1727 - 1758 CODEN: CMCHE7; ISSN: 0929-8673, 2001, XP009056485
- GRAUL, A. ET AL: "HMR-3647, an antimicrobial ketolide" DRUGS OF THE FUTURE , 23(6), 591-597 CODEN: DRFUD4; ISSN: 0377-8282, 1998, XP000909275
- DENIS, ALEXIS ET AL: "Synthesis and antibacterial activity of HMR 3647, a new ketolide highly potent against erythromycin-resistant and susceptible pathogens" BIOORGANIC & MEDICINAL CHEMISTRY LETTERS , 9(21), 3075-3080 CODEN: BMCLE8; ISSN: 0960-894X, 1999, XP004181010

## Description

### Field of the invention

The present invention relates to the process for the preparation of Telithromycin of formula (I) and its pharmaceutically acceptable salts. 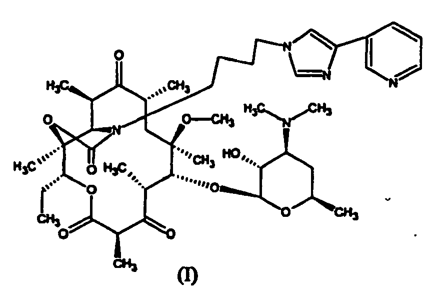 Telithromycin of formula (I) has an antibiotic activity.

### Background of the invention

Macrolide compounds are known for anti bacterial activity. The rapid development of antibiotic resistance among the major respiratory pathogens has created a serious problem for the effective management of respiratory tract infections. There is a great medical need for new antibiotics to address the problem of antibiotic resistance. Under these circumstances, several novel series of macrolides with a common C-3 ketone group were recently introduced, which are collectively known as ketolides.

Ketolides represent a novel class of macrolide antibiotics that have received much attention recently on account of their excellent activity against resistant organisms. Most ketolides are derivatives of erythromycin, a potent and safe antibiotic widely prescribed for the treatment of respiratory tract infections for more than four decades. Ketolides are 14-membered ring macrolide derivatives characterized by a keto group at the C-3 position [Curr. Med. Chem. - Anti-Infective Agents, 2002, 1, 15-34]. Several Ketolide compounds are under clinical investigation. However, Telithromycin of Formula (I) is the first agent to receive approvable status in this class of drugs.

US patent 5635485 discloses several ketolide compounds, which are prepared by condensing compounds of Formula (II) with amine of formula (III) in a solvent for prolonged hours to yield compound of formula (IV), followed by removal of protecting group Z' at 2' position by hydrolysis as shown in Scheme -1. Furthermore, Formula (II) has been prepared by following US 5527780. 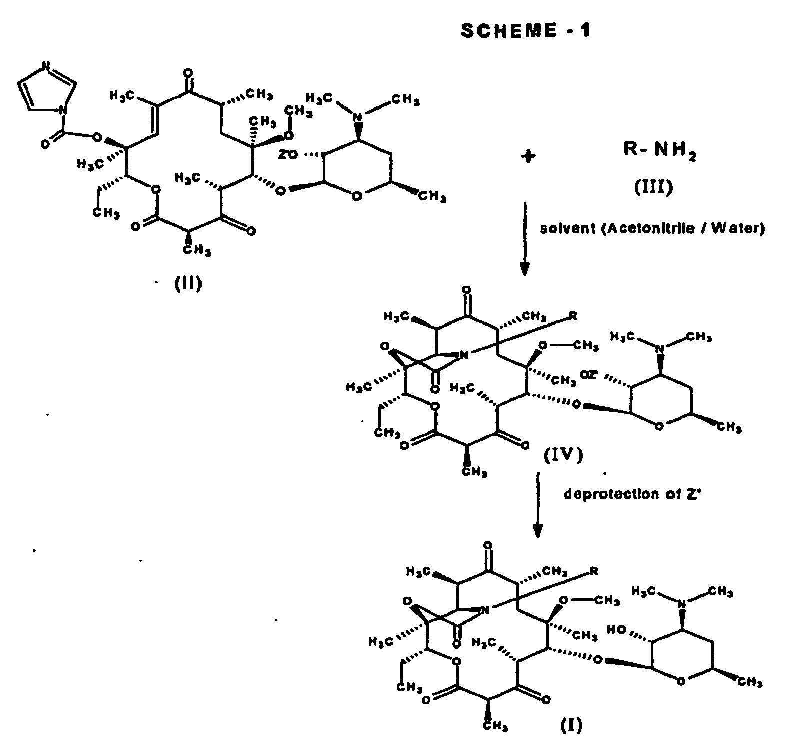 wherein, definition of R and Z' are as defined in above referred patent.

Accordingly, Telithromycin is prepared by condensing compound of formula (II) with amine of formula (III), where in 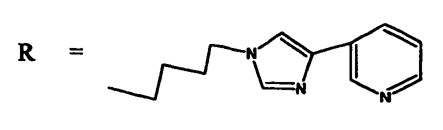 followed by removing the protecting group to yield Telithromycin of formula (I). The preparation of formula II is disclosed in Current Medicinal chemistry, 2001, Vol. 8, 1727-1758. The process described in US 5635485 suffers several drawbacks such as
(i) Condensation of formula II with formula III is cumbersome and it is very difficult to remove unreacted reagents and impurities formed during the reaction.
(ii) The isolation and purification of the desired compound of Formula (I) cannot be done without laborious column chromatography, which is not viable at commercial production level.

Current Medicinal Chemistry, 2001, Vol. 8, 1727-1758 also describes the process for the preparation of various ketolides, including Telithromycin in which Clarithromycin (formula V) is reacted with hydrochloric acid to remove cladinose ring at C-3 position (formula VI) followed by selective acetylation of the 2'-hydroxy group in formula VI and selective oxidation of the 3-hydroxy group generated ketolide of formula VII. Further, 11-hydorxy group of compound of formula (VII) is selectively mesylated followed by base induced β- elimination to furnish α,β-unsaturated ketone (formula VIII). The compound of formula (VIII) is further treated with sodium hydride and carbonyldiimidazole to form 12-O-acyl imidazole of formula (II), which upon stereoselective cyclization with (4-(3-pyridinyl)-imidazol-1-yl)-butylamine and subsequent deprotection of the 2'-hydroxy group gives Telithromycin of Formula (I). This process is outlined in following SCHEME - 2

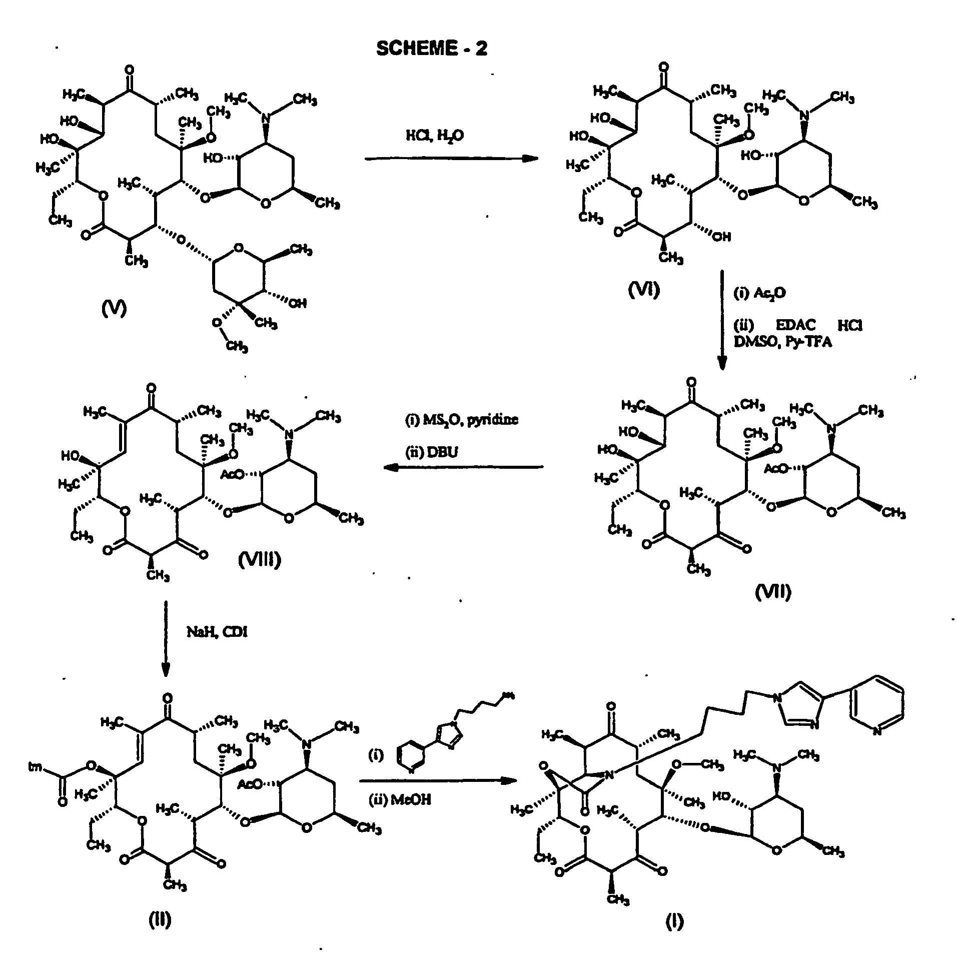

However, this process also consists of several difficulties as explained above and moreover other difficulties such as use of pyrophoric material like NaH, which is hazardous and extremely difficult to handle at the plant scale.

In light of the above difficulties for the preparation of Telithromycin, this process is not suitable for commercial production level.

### Objects of the invention

Therefore the basic object of this invention is to provide a process for the preparation of Telithromycin.

Another object of the present invention is to provide a process for the preparation of Telithromycin, which would be high yielding, cost effective, easy to operate at industrial scale and would not involve the use of moisture sensitive, pyrophoric compounds such as sodium hydride.

Another object of the present invention is to provide a process of manufacture of Telithromycin, which lead to the removal of reagents and side products by intermediate crystallization. Thus, isolation of final product enabling good yield and purity, without column chromatography.

A further objective of the invention is to provide a process of manufacture of Telithromycin that would involve selective mild reaction conditions.

A further object of the invention is to provide a process of manufacture of Telithromycin that would be industrially feasible.

Still, another object of the present invention is to provide a novel compound of formula (Xa), (XIa), (XIIa) and (XIIIa), which are useful as an intermediate for the preparation of Telithromycin (I).

### Summary of the invention:

Present invention provides the process for the preparation of Telithromycin of formula (I) or its pharmaceutically acceptable salts 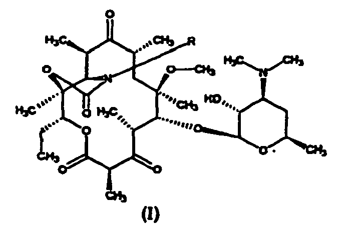 where, R is 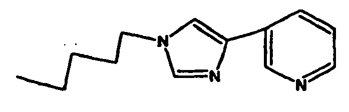 comprising
(a) reacting compound of formula (IX) 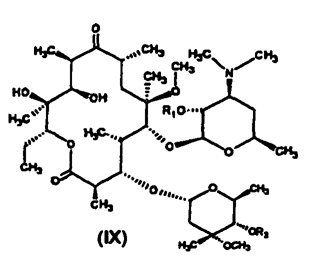 with carbonyldiimidazole in presence of polar solvent and base to obtain the compound of formula (X) 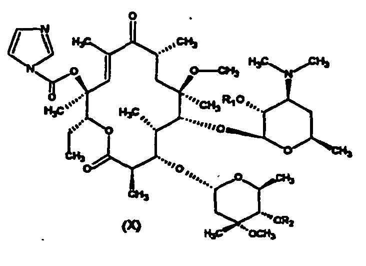 where R₁ and R₂ are same or different protecting groups represented by 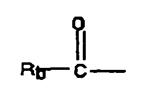 R_{b} is C₁ to C₁₀ alkyl group or aryl group, preferably R_{b} is C₁ - C₄ alkyl group, aryl represents substituted or unsubstituted phenyl group; more preferably R₁ and R₂ are same or different acetyl, benzyl or benzoyl group
(b) condensing the compound of formula (X) with R-NH₂ in suitable polar solvent to give compounds of formula (XI). 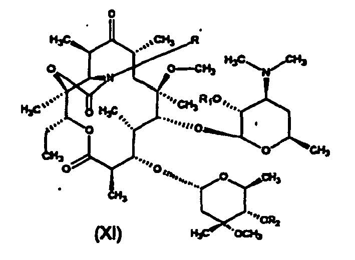 where R is as defined above and R₁ and R₂ are also same as defined above.
(c) treating the compound of formula (XI) with acid to obtain compound of formula (XII) 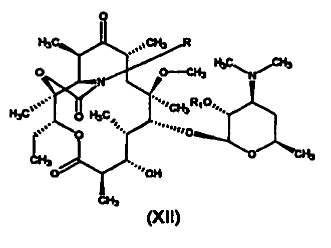
(d) oxidising the resulting compound of formula (XII) in presence of oxidizing agent to give compounds of formula (XIII) 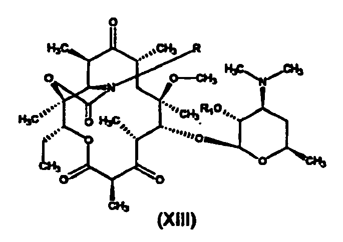
(e) removing the protecting group at 2' position of formula (XIII) by treating with alcohol to give Telithromycin of formula (I)

The reaction scheme for the preparation of Telithromycin (I) is as shown in Scheme-3 hereunder:

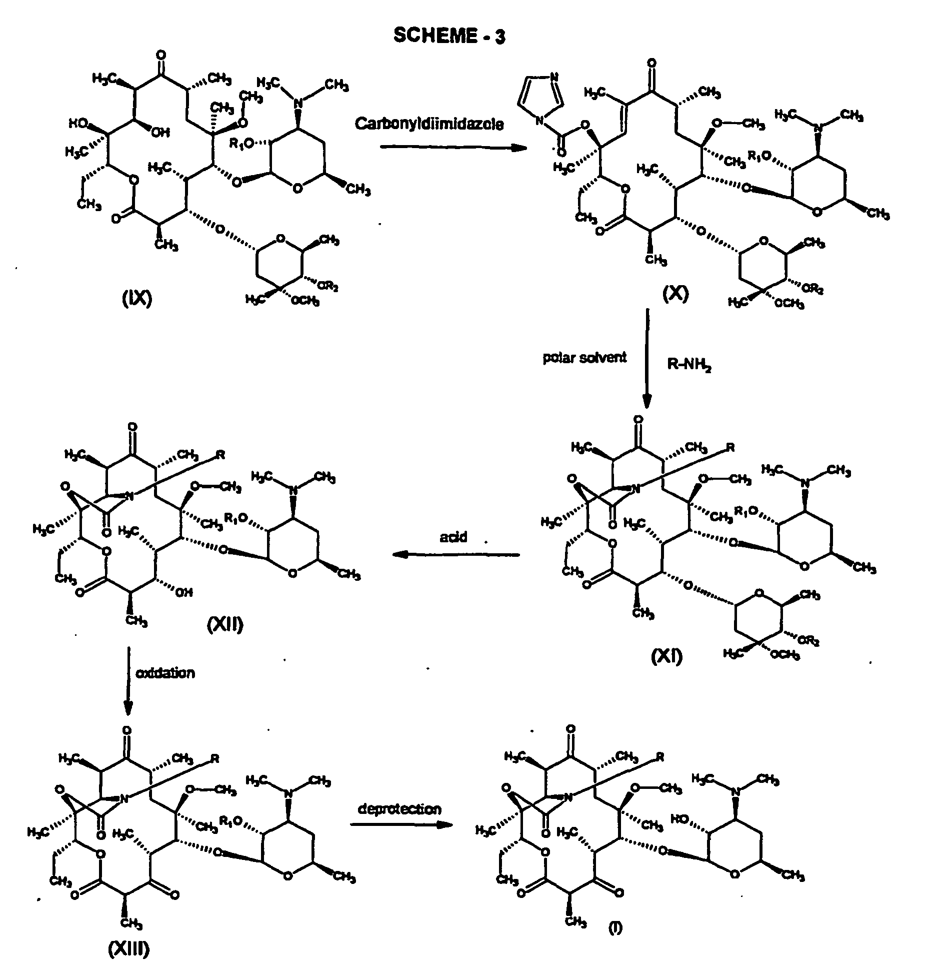

Alternatively, the process for the preparation of Telithromycin of formula (I) or its pharmaceutically acceptable salts, which comprises
(a) reacting compound of formula (IX) 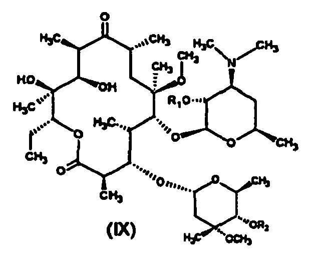 with carbonyldiimidazole in presence of polar solvent and base to obtain the compound of formula (X) 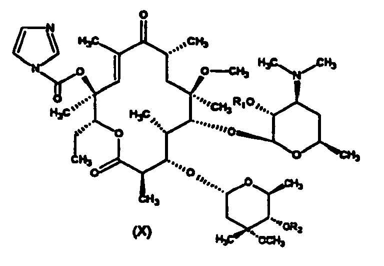 where R₁ and R₂ are same or different protecting groups represented by 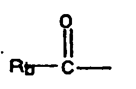 R_{b} is C₁ to C₁₀ alkyl group or aryl group, preferably R_{b} is C₁ - C₄ alkyl group, aryl represents substituted or unsubstituted phenyl group; more preferably R₁ and R₂ are same or different acetyl, benzyl or benzoyl group
(b) condensing the compound of formula (X) with R-NH₂ in suitable polar solvent to give compounds of formula (XI). 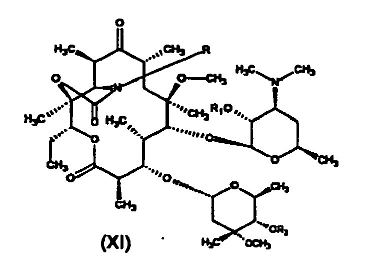 where R is as defined above and R₁ and R₂ are also same as defined above.
(c) treating the compound of formula (XI) with acid to obtain compound of formula (XII) 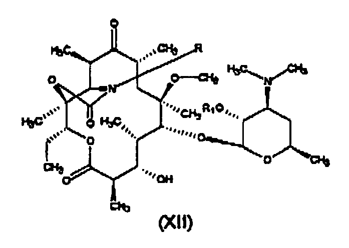
(f) treating compounds of formula (XII) as received from step (c) with alcohol to give compounds of formula (XIV) 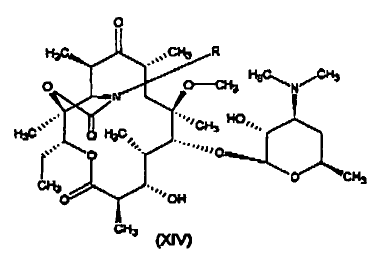
(g) selective oxidization of resulting compounds of formula (XIV) of step (f) in the presence of oxidizing agent to form desired ketolide compound of formula (I)

Optionally the compound of formula XIV may be crystallized using a polar solvent selected from acetone, alcohol, ethyl acetate, preferably acetone.

The reaction scheme followed is as shown in Scheme-4 hereunder:

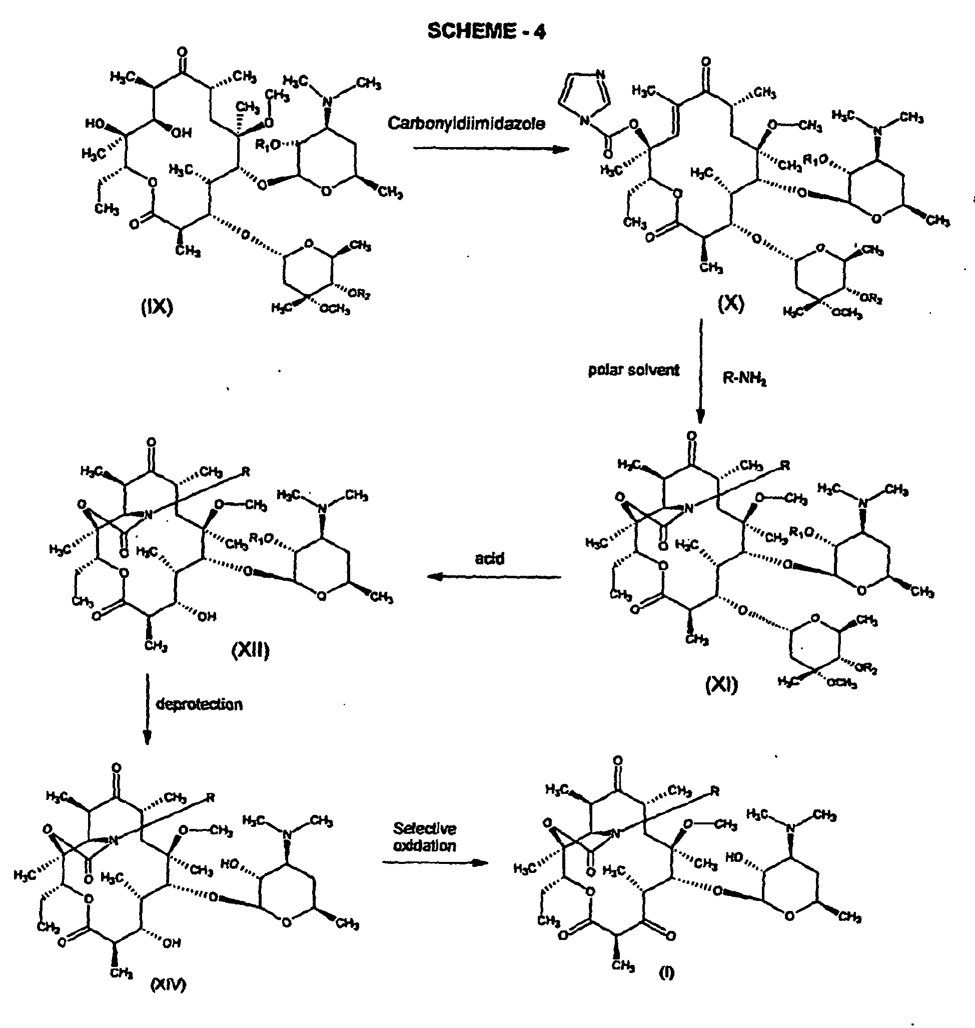

Also, the present invention provides the novel compounds of formula (Xa), (XIa), (XIIa), (XIIIa), where R₁ and/or R₂ is benzoly (Bz) as stated in the above compounds of formula (X), (XI), (XII) and (XIII) respectively. 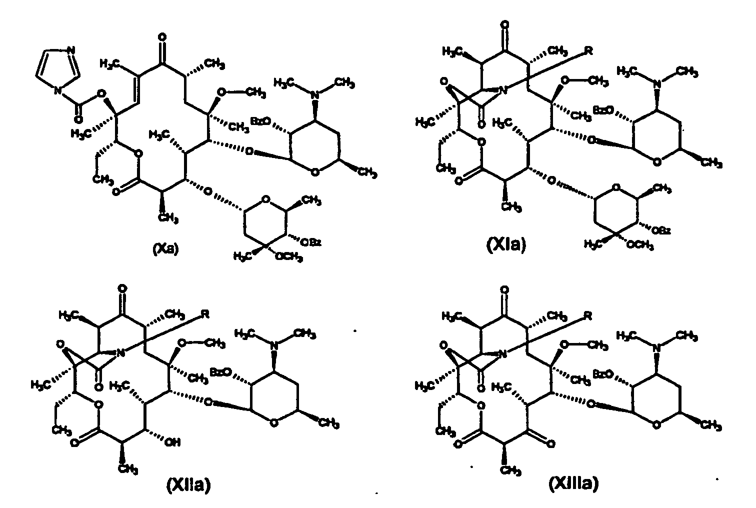 where is R is as defined above.

### Detailed description of the invention

The polar solvent use in step (a) is selected from dimethylformamide, tetrahydrofuran, acetonitrile and mixtures thereof.

The base used in step (a) is selected from DBU, Triethylamine, diisopropylethylamine.

The polar solvent used in step (b) is a polar aprotic solvent or polar protic solvent The solvent is selected from the group comprises of methanol, ethanol, isopropanol, n-propanol, n-butanol, iso butyl alcohol, tert-butyl alcohol, methoxyethanol, ethoxyethanol, pentanol, neo-pentyl alcohol, t-pentyl alcohol, cyclohexanol, ethylene glycol, propylene glycol, benzyl alcohol, phenol, glycerol, dimethylformamide (DMF), dimethylacetamide (DMAC), 1,3-dimethyl-3,4,5,6-tetrahydro-2(1H)-pyrimidinone (DMPU), 1,3-dimethyl-2-imidazolidinone (DMI), N-methylpyrrolidinone (NMP), formamide, N-methylacetamide, N-methylformamide, acetonitrile, dimethylsulfoxide, propionitrile, ethyl formate, methyl acetate, hexachloroacetone, HMPA, HMPT, acetone, ethyl methyl ketone, ethyl acetate, isopropyl acetate, t-butyl acetate, sulfolane, N,N-dimethylpropionamide, nitromethane, nitrobenzene, teteahydrofuran (THF), dioxane, water, polyethers or mixtures thereof.

Acid referred in step (c) is organic acid or inorganic acid selected from group comprising of hydrochloric acid, hydrobromic acid, sulfuric acid, nitric acid, phosphoric acid, perchloric acid or hydrofluoric acid. The preferred acid is hydrochloric acid. The reaction step (c) is carried out in a solvent selected from water, polar organic solvent such as alcohols selected from methanol, ethanol, isopropanol, n-propanol, tert-butanol, n-butanol or mixture there of.

The said step is carried out at 0° to 70° C and more preferably at 20 to 60° C.

The oxidation in step (d) is carried out by using the commonly used oxidising reagents such as Corey- Kim oxidation method, Dess- Martins reagent, Pfitzner moffat method or modifications thereof or with dimethyl sulfoxide in presence of oxalyl chloride or phosphorous pentoxide or p-Toluene sulfonyl chloride or acetic anhydride or N-chlrosuccinimide. The oxidation can also be carried out by Manganese or chromium or selenium reagents, tert-amine oxides or by any above oxidant in presence of phase transfer catalyst.

The alcohol referred in step (e) is selected from group comprising of methanol, ethanol, n-propanol, isopropanol, tert-butanol, n-butanol or mixtures there of. The preferred alcohol is methanol.

The reaction step (e) is carried out at a temperature of 0 to 100° C and preferably at 20 to 70° C. The step (e) can also be carried out in presence of mineral acid selected from HCl or H₂SO₄.

Alternative process for the preparation of Telithromycin, the reaction step (f) is carried out in presence of alcohol to give compounds of formula (XIV). The alcohol in step (f) is selected from group comprising of methanol, ethanol, n-propanol, isopropanol, tert-butanol, n-butanol or mixtures there of. The preferred alcohol is methanol.

The reaction step (f) is carried out at a temperature of 0 to 100° C and preferably at 20 to 70° C. The step (f) can also be carried out in presence of mineral acid selected from HCl or H₂SO₄.

The compound formula (XIV) is selectively oxidized in step (g) to give Telithromycin of formula (I). The said oxidation is carried out using Corey- Kim Martins reagent, Pfitzner moffat method or modifications thereof or with dimethyl sulfoxide in presence of oxalyl chloride or phosphorous pentoxide or p-Toluene sulfonyl chloride or acetic anhydride or N-chlrosuccinimide.

According to another embodiment of the present invention, there is provided a process for the preparation of novel compound of formula (Xa), (XIa), (XIIa), (XIIIa).

The process for the preparation of compound of formula (XIIIa), which comprises
(a') reacting 2',4"-di-O-benzoyl-6-O-methylerythromycin A compound of formula (IXa) 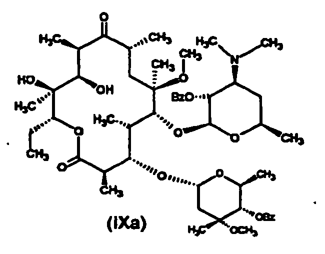 with carbonyldiimidazole in presence of a polar solvent and base to obtain 10,11-anhydro-2',4"-di-O-benzoyl-12-O-imidazolylcarbonyl-6-O-methylerythromycin A (Xa) 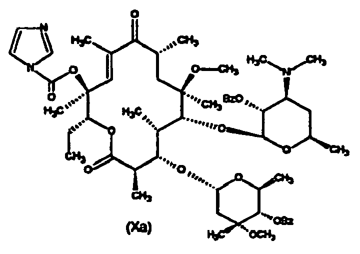
(b') condensing the compounds of formula (Xa) with R-NH₂ in a suitable polar solvent in presence of base to obtain 2',4"-di-O-benzoyl-11-amino-11-N-[4-[4-(3-pyndyl)imidazol-1-yl]butyl]-11-deoxy-6-O-methylerythromycin A 11,12-cyclic carbamate of formula (XIa) 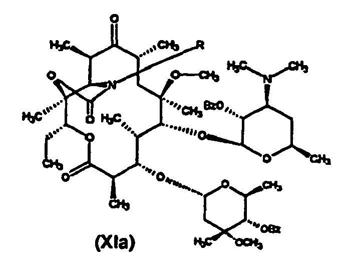
(c') treating the obtained compound formula (XIa) with an acid to give 2'-O-benzoyl-11-amino-11-N-[4-[4-(3-pyridyl)imidazol-1-yl]butyl]-11-deoxy-5-O-desosaminyl-6-O-methylerythronolide A 11,12-cyclic carbamate of formula (XIIa) 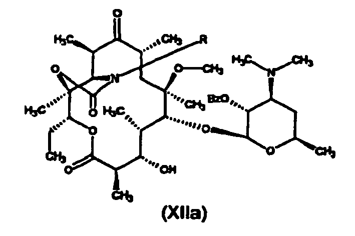
(d') oxidizing the resulting compounds of formula (XIIa) in presence of oxidizing agent to obtain the compound of formula (XIIIa)

The compound of formula (XIIIa) obtained by above process can be converted to Telithromycin (I) or its pharmaceutically acceptable salts by treating it with alcohols.

The polar solvent used in step (a') is selected from dimehtylformamide, tetrahydrofuran, acetonitrile and mixtures thereof.

The reaction step (a') is carried out in presence of base selected from DBU, triethylamine, diisopropylethylamine.

Polar solvent used in step (b') is selected from group comprising of methanol, ethanol, isopropanol, n-propanol, n-butanol, iso butyl alcohol, tert-butyl alcohol, methoxyethanol, ethoxyethanol, pentanol, neo-pentyl alcohol, tert-pentyl alcohol, cyclohexanol, ethylene glycol, propylene glycol, benzyl alcohol, phenol, glycerol, dimethylformamide (DMF), dimethylacetamide (DMAC), 1,3-dimethyl-3,4,5,6-tetrahydro-2(1H)-pyrimidinone (DMPU), 1,3-dimethyl-2-imidazolidinone (DMI), N-methylpyrrolidinone (NMP), formamide, N-methylacetamide, N-methylformamide, acetonitrile, dimethylsulfoxide, propionitrile, ethyl formate, methyl acetate, hexachloroacetone, HMPA, HMPT, acetone, ethyl methyl ketone, ethyl acetate, isopropyl acetate, t-butyl acetate, sulfolane, N,N-dimethylpropionamide, nitromethane, nitrobenzene, tetrahydrofuran (THF), dioxane, water, polyethers or mixtures thereof. The preferred solvent is dimethylformamide or acetonitrile.

The reaction step (b') is carried out in presence of base selected from DBU, triethylamine, diisopropylethylamine. The said step is preferably carried out at a temperature 30°C to 60° C.

Acid referred in step (c') is organic acid or inorganic acid selected from group comprising of hydrochloric acid, hydrobromic acid, sulfuric acid, nitric acid, phosphoric acid, perchloric acid or hydrofluoric acid. The preferred acid is hydrochloric acid. The reaction step (c') is carried out in a solvent selected from water, polar organic solvent such as alcohols selected from methanol, ethanol, isopropanol, n-propanol, tert-butanol, n-butanol or mixture there of.

The said step is carried out at 0 to 70° C and more preferably at 20 to 60° C.

The oxidation in step (d') is carried out by using the commonly used oxidising reagents such as Corey- Kim oxidation method, Dess- Martin reagent, Pfitzner Moffat method or modifications thereof or with dimethyl sulfoxide in presence of oxalyl chloride or phosphorous pentoxide or p-Toluene sulfonyl chloride or acetic anhydride or N-chlorosuccinimide. The oxidation can also be carried out by Manganese or chromium or selenium reagents, tert-amine oxides or by any above oxidant in presence of phase transfer catalyst.

Another embodiment of the present invention, the process for the preparation of Telithromycin of formula (I) comprises
(i) reacting 2'4"-di-O-acetyl-6-O-methylerythromycin A (obtained as indicated in example 1(1) of U.S. patent US 5591837) with carbonyldiimidazole in presence of polar solvent and base to give 10,11-Anhydro-2'4"-di-O-acetyl-12-O-imidazolyl carbonyl-6-O-methylerythromycin A, the polar solvent being selected from Dimehtylformamide, Tetrahydrofuran, Acetonitrile and mixtures thereof and the base selected from DBU, Triethylamine, diisopropylethylamine.
(ii) condensing 10,11-anhydro-2'4"-di-O-acetyl-12-O-imidazolyl carbonyl-6-O-methylerythromycin A with 4-[4-(3-pyridyl)imidazol-1-yl]butaneamine in a polar solvent at 5° to 120°C to give 2',4"-di-O-acetyl-11-amino-11-N-[4-[4-(3-pyridyl)imidazol-1-yl]butyl]-11-deoxy-6-O-methylerythromycin A 11,12-cyclic carbamate, said polar solvent is polar aprotic solvent or polar protic solvent;
(iii) reacting 2',4"-di-O-acetyl-11-amino-11-N-[4-[4-(3-pyridyl)imidazol-1-yl]butyl]-11-deoxy-6-O-methylerythromycin A 11,12-cyclic carbamate with acid at 0°C to 100°C by removal cladinose ring at C-3 position to obtain 2'-O-acetyl-11-amino-11-N-[4-[4-(3-pyridyl) imidazol-1-yl] butyl]-11-deoxy-5-O-desosaminyl-6-O-methylerythronolide A 11,12-cyclic carbamate.
(iv) Further, 2'-O-acetyl-11-amino-11-N-[4-[4-(3-pyridyl) imidazol-1-yl] butyl]-11-deoxy-5-O-desosaminyl-6-O-methylerythronolide A 11,12-cyclic carbamate is oxidized at C-3 position to give 2'-O-acetyl-11-amino-11-N-[4-[4-(3-pyridyl) imidazol-1-yl] butyl]-11-deoxy-3-oxo-5-O-desosaminyl-6-O-methylerythronolide A 11,12-cyclic carbamate;
(v) 2'-O-acetyl-11-amino-11-N-[4-[4-(3-pyridyl) imidazol-1-yl] butyl]-11-deoxy-3-oxo-5-O-desosaminyl-6-O-methylerythronolide A 11,12-cyclic carbamate is further treated with alcohols to remove protecting group at 2' position to give Telithromycin of formula (I).

In step (ii) of the process the polar solvents is selected form the group comprises of methanol, ethanol, n-propanol, isopropanol, n-butanol, isobutyl alcohol, tert-butyl alcohol, methoxyethanol, ethoxyethanol, pentanol, neo-pentyl alcohol, t-pentyl alcohol, cyclohexanol, ethylene glycol, propylene glycol, benzyl alcohol, phenol, glycerol, dimethylformamide (DMF), dimethylacetamide (DMAC), 1,3-dimethyl-3,4,5,6-tetrahydro-2(1H)-pyrimidinone (DMPU), 1,3-dimethyl-2-imidazolidinone (DMI), N-methylpyrrolidinone (NMP), formamide, N-methylacetamide, N-methylformamide, acetonitrile, dimethylsulfoxide, propionitrile, ethyl formate, methyl acetate, hexachloroacetone, HMPA, HMPT, acetone, ethyl methyl ketone, ethyl acetate, isopropyl acetate, t-butyl acetate, sulfolane, N,N-dimethylpropionamide, nitromethane, nitrobenzene, tetrahydrofuran (THF), dioxane, polyethers or water or mixtures thereof.

The preferred polar solvent is dimethylformamide (DMF) and acetonitrile.

The most preferred solvent is dimethylformamide.

The reaction step is carried out at 5 to 120° C. Preferably step (i) can be carried out at 30 to 60° C. The reaction also can be carried out in water or the mixture of water and organic solvents (as mentioned above).

The ratio of substrate to amine is 1: 3 mole and the preferably ratio is 1:2 mole.

In step (iii) of the process the acid is selected from organic acid or inorganic acid or mixtures thereof. Inorganic acid can be mineral acid selected from hydrochloric acid, hydrobromic acid, sulfuric acid, nitric acid, phosphoric acid and perchloric acid, hydrofluoric acid. The acid is preferably hydrochloric acid.

The solvent is selected from the group comprising water or polar organic solvents like alcohols or mixtures thereof. The preferred solvents can be water, methanol, ethanol, iso propanol, n- butanol, tert-butanol or mixtures thereof.

The reaction step is carried out at 0 to 100° C and more preferably 20°C to 60°C for 6 to 48 hrs.

In step (iv) of the process the oxidation can be carried out by way of Corey- Kim oxidation method, Dess- Martins reagent, Pfitzner Moffat method or modifications thereof or with dimethyl sulfoxide in presence of oxalyl chloride or phosphorous pentoxide or p-Toluene sulfonyl chloride or acetic anhydride or N-chlorosuccinimide. The oxidation can also be carried out by Manganese or chromium or selenium reagents, tert-amine oxides or any above oxidant in presence of phase transfer catalyst.

In step (v) of the process the alcohol is selected from the group comprising of methanol, ethanol, n-propanol, isopropanol, n-butanol, tert-butanol or mixtures there of or with water at 0°C to 100°C to give desired ketolide compounds of formula (I).

Alternatively, pure and commercially viable process for the preparation of Telithromycin is by carrying out first deprotection of 2'-O-acetyl-11-amino-11-N-[4-[4-(3-pyridyl) imidazol-1-yl] butyl]-11-deoxy-5-O-desosaminyl-6-O-methylerythronolide A 11,12-cyclic carbamate as obtained in step (iii) purification and then oxidation of the resultant compound.

The detail process is described as below:
(vi) 2'-O-acetyl-11-amino-11-N-[4-[4-(3-pyridyl) imidazol-1-yl] butyl]-11-deoxy-5-O-desosaminyl-6-O-methylerythronolide A 11,12-cyclic carbamate is treated with an alcohol at 0 to 70°C or with water at 0° to 100°C to remove acetyl protecting group and form 2'-hydroxy-11-amino-11-N-[4-[4-(3-pyridyl) imidazol-1-yl] butyl]-11-deoxy-5-O-desosaminyl-6-O-methylerythronolide A 11,12-cyclic carbamate or with water at 0°C to 100°C to give desired compound of formula (XIV) the compound of formula (XIV) is crystallised by using polar solvent.
(vii) 2'-hydroxy -11-amino-11-N-[4-[4-(3-pyridyl) imidazol-1-yl] butyl]-11-deoxy-5-O-desosaminyl-6-O-methylerythronolide A 11,12-cyclic carbamate is selectively oxidized at C-3 position to form Telithromycin of formula (I).

In step (vi) the alcohol is selected from the group comprising of methanol, ethanol, n-propanol, isopropanol, n-butanol, tert-butanol or mixtures thereof. In the said step the polar solvent is selected from acetone or alcohol or ethyl acetate or mixture thereof. The solvent used for the crystallisation of formula (XIV) is preferably acetone.

In step (vii) the oxidation is carried out by way of Corey- Kim oxidation method, Dess- Martin reagent, Pfitzner Moffat method or modifications thereof or with dimethyl sulfoxide in presence of oxalyl chloride or phosphorous pentoxide or p-Toluene sulfonyl chloride or acetic anhydride. The oxidation can also be carried out by manganese or chromium or selenium reagents, tert-amine oxides or any above oxidant in presence of phase transfer catalyst.

The process of the present invention is described by the following examples, which are illustrative only and should not be construed so as to limit the scope of the invention in any manner.

### EXAMPLES:

### Example 1: Preparation of 10,11-Anhydro-2'4"-di-O-acetyl-12-O-imidazolyl carbonyl-6-O-methylerythromycin A.

Mix 10 gm of 2',4"-di-O-acetyl-6-O-methylerythromycin A, 10 gm of carbonyldiimidazole, 40 ml dimethylformamide and 4 ml DBU at room temperature. The solution was cleared. The clear solution was stirred for 3 hrs. The reaction mixture was quenched with water (400ml). The solid was filtered and washed with water. The wet solid was dissolved in dichloromethane and the organic layer was separated. The solvent was removed under vacuum. Add diisopropylether (40 ml) and the reaction mixture was stirred for half an hour. The solid was filtered and washed with diisopropylether (2 X 5 ml). The solid was dried at room temperature to give 9.0 gm of 10,11-Anhydro-2'4"-di-O-acetyl-12-O-imidazolylcarbonyl-6-O- methyl erythromycin A.

### Example 2: Preparation of 2',4"-di-O-acetyl-11-amino-11-N-[4-[4-(3-pyridyl)imidazol-1-yl]butyl]-11-deoxy-6-O-methylerythromycin A 11,12-cyclic carbamate.

20gm of 10,11-Anhydro-2'4"-di-O-acetyl-12-O-imidazolylcarbonyl-6-O- methyl erythromycin A was added in 9.6 gm of 4-[4-(3-pyridyl) imidazol-1-yl] butanamine and 100ml Dimethyl formamide and stirred at 50 °C for 18 hours. The reaction mixture was then diluted with water and stirred for 30min. The precipitated solid was filtered and washed with water. Further, it was dried at 50 °C under vacuum to give 18gm of 2', 4"-di-O-acetyl-11-amino-11-N-[4-[4-(3-pyridyl)imidazol-1-yl]butyl]-11-deoxy-6-O-methylerythromycin A 11,12-cyclic carbamate.

### Example 3: Preparation of 2'-O-acetyl-11-amino-11-N-[4-[4-(3-pyridyl) imidazol-1-yl] butyl]-11-deoxy-5-O-desosaminyl-6-O-methylerythronolide A 11,12-cyclic carbamate.

23g of 2',4"-di-O-acetyl-11-amino-11-N-[4-[4-(3-pyridyl)imidazol-1-yl]butyl]-11-deoxy-6-O-methylerythromycin A 11,12-cyclic carbamate was dissolved in solution of 23ml concentrated hydrochloric acid and 230ml water. The mixture was stirred at ambient temperature for 12 hours. The reaction mixture was then basified with sodium hydroxide when a white solid was obtained. The solid was filtered and washed with water. Drying at ambient temperature afforded 16gm of 2'-O-acetyl-11-amino-11-N-[4-[4-(3-pyridyl) imidazol-1-yl] butyl]-11-deoxy-5-O-desosaminyl-6-O-methylerythronolide A 11,12-cyclic carbamate. The product can be used in the next step without further purification.

### Example 4: Preparation of 2'-O-acetyl-11-amino-11-N-[4-[4-(3-pyridyl) imidazol-1-yl] butyl]-11-deoxy-3-oxo-5-O-desosaminyl-6-O-methylerythronolide A 11, 12-cyclic carbamate

15g of 2'-O-acetyl-11-amino-11-N-[4-[4-(3-pyridyl) imidazol-1-yl] butyl]-11-deoxy-5-O-desosaminyl-6-O-methylerythronolide A 11,12-cyclic carbamate was dissolved in 150 ml dichloromethane and 22.5g of Dess Martin reagent was added in one lot. The mixture was stirred at ambient temperature for 1 hour. Further, added the mixture of 260 ml saturated sodium bicarbonate solution and saturated sodium thiosulfate solution and stirred the mixture for 20 min. Filtered off the formed solid precipitate and separated the organic layer. Washed the organic layer with water, dried over sodium sulfate and distilled off the solvent under vacuum to give 13gm of 2'-O-acetyl-11-amino-11-N-[4-[4-(3-pyridyl) imidazol-1-yl] butyl]-11-deoxy-3-oxo-5-O-desosaminyl-6-O-methylerythronolide A 11,12-cyclic carbamate.

### Example 5: Preparation of 2'-O-acetyl-11-amino-11-N-[4-[4-(3-pyridyl) imidazol-1-yl] butyl]-11-deoxy-3-oxo-5-O-desosaminyl-6-O-methylerythronolide A 11,12-cyclic carbamate

10g of 2'-O-acetyl-11-amino-11-N-[4-[4-(3-pyridyl) imidazol-1-yl] butyl]-11-deoxy-5-O-desosaminyl-6-O-methylerythronolide A 11,12-cyclic carbamate was dissolved in 100 ml dichloromethane. Dimethyl sulfoxide (16.6 ml), Cyclohexyl dimethyl amino propyl carbodimide Hydro chloride (25.0g) and Pyridine HCl (12.1 gm) was added in one lot. The mixture was stirred at ambient temperature (20-30°C) for 6 hour. Further, 500 ml water was added and stirred the mixture for 30 min. The organic layer was separated and washed with water, dried over sodium sulfate and distilled off the solvent under vacuum to give 7.05 gm of 2'-O-acetyl-11-amino-11 N-[4-[4-(3-pyridyl) imidazol-1-yl] butyl]-11-deoxy-3-oxo-5-O-desosaminyl-6-O-methylerythronolide A 11,12-cyclic carbamate.

### Example 6: Preparation of 2'-O-acetyl-11-amino-11-N-[4-[4-(3-pyridyl) imidazol-1-yl] butyl]-11-deoxy-3-oxo-5-O-desosaminyl-6-O-methylerythronolide A 11,12-cyclic carbamate

N-Chloro succinimide (4.68 gm) was charged to the reaction vessel under nitrogen atmosphere and Dichloromethane (200 ml) was added slowly. The reaction masses was cooled to 0°C, Dimethyl sulfide (3.5 ml) was added slowly and continue the stirring at same temperature for half an hour. The reaction mass was cooled to -25°C and 2'-O-acetyl-11-amino-11-N-[4-[4-(3-pyridyl) imidazol-1-yl] butyl]-11-deoxy-5-O-desosaminyl-6-O-methylerythronolide A 11,12-cyclic carbamate (10g) dissolved in 50 ml dichloromethane was added slowly. The mixture was stirred at -25°C temperature for 2 hour. At the same temperature Diisopropyletheyl amine (0.6ml) was added and the reaction mixture was stirred for an hour. Water (500 ml) was added and stirred the mixture for 30 min. The organic layer was separated and washed with water, dried over sodium sulfate and distilled off the solvent under vacuum to give 7.5 gm of 2'-O-acetyl-11-amino-11-N-[4-[4-(3-pyridyl) imidazol-1-yl] butyl]-11-deoxy-3-oxo-5-O-desosaminyl-6-O-methylerythronolide A 11,12-cyclic carbamate.

### Example 7: 11-Amino-11-N-[4-[4-(3-pyridyl) imidazol-1-yl]butyl-11-deoxy-3-oxo-5-O-desosaminyl-6-O-methylerythronolide A 11,12-cyclic carbamate [Telithromycin] (I)

A solution for 10g of 2'-O-acetyl-11-amino-11-N-[4-[4-(3-pyridyl) imidazol-1-yl] butyl]-11-deoxy-3-oxo-5-O-desosaminyl-6-O-methylerythronolide A 11,12-cyclic carbamate in 100ml methanol was stirred at ambient temperature for 16 hours. Further, solvent was distilled off under vacuum and stirring remain solid with 50ml diisoproyl ether to gave 7gm of desired compound 11-Amino-11-N-[4-[4-(3-pyridyl) imidazol-1-yl]butyl-11-deoxy-3-oxo-5-O-desosaminyl-6-O-methylerythronolide A 11,12-cyclic carbamate [Telithromycin] (I).

### Example 8: 11-Amino-11-N-[4-[4-(3-pyridyl) imidazol-1-yl]butyl-11-deoxy 3-oxo-5-O-desosaminyl-6-O-methylerythronolide A 11,12-cyclic carbamate [Telithromycin] (I)

A solution for 10g of 2'-O-acetyl-11-amino-11-N-[4-[4-(3-pyridyl) imidazol-1-yl] butyl]-11-deoxy-3-oxo-5-O-desosaminyl-6-O-methylerythronolide A 11,12-cyclic carbamate in 100ml *Isopropanol* was stirred at ambient temperature for 24 hours. Further, solvent was distilled off under vacuum and equilibrating remain solid with 50ml diisoproyl ether to gave 7gm of desired compound 11-Amino-11-N-[4-[4-(3-pyridyl) imidazol-1-yl]butyl-11-deoxy-3-oxo-5-O-desosaminyl-6-O-methylerythronolide A 11,12-cyclic carbamate [Telithromycin] (I).

### Example 9: 2'-hydroxy -11-amino-11-N-[4-[4-(3-pyridyl) imidazol-1-yl] butyl]-11-deoxy-5-O-desosaminyl-6-O-methylerythronolide A 11,12-cyclic carbamate.

10g of 2'-O-acetyl-11-amino-11-N-[4-[4-(3-pyridyl) imidazol-1-yl] butyl]-11-deoxy-5-O-desosaminyl-6-O-methylerythronolide A 11,12-cyclic carbamate in 100 ml methanol was stirred at reflux temperature for 6 hours. The solvent was then distilled off under vacuum to give crude product as white foam. Then, the crude product was purified by refluxing in 20 ml of acetone followed by 1 hour stirring at 10°C. Filtered off the solution and washed the solid and with 2×5 ml of chilled acetone to gave 8.0gm 2'-hydroxy -11-amino-11-N-[4-[4-(3-pyridyl) imidazol-1-yl] butyl]-11-deoxy-5-O-desosaminyl-6-O-methylerythronolide A 11,12-cyclic carbamate

### Example 10: 2'-hydroxy -11-amino-11-N-[4-[4-(3-pyridyl) imidazol-1-yl] butyl]-1 1-deoxy-5-O-desosaminyl-6-O-methylerythronolide A 11,12-cyclic carbamate.

10g of 2'-O-acetyl-11-amino-11-N-[4-[4-(3-pyridyl) imidazol-1-yl] butyl]-11-deoxy-5-O-desosaminyl-6-O-methylerythronolide A 11,12-cyclic carbamate in 100 ml *Isoproapnol* was stirred at reflux temperature for 18 hours. The solvent was then distilled off under vacuum to give crude product as white foam. Then, the crude product was purified by refluxing in 20 ml of acetone followed by 1 hour stirring at 10°C. Filtered off the solution and washed the solid and with 2x5 ml of chilled acetone to gave 8.0gm 2'-hydroxy -11-amino-11-N-[4-[4-(3-pyridyl) imidazol-1-yl] butyl]-11-deoxy-5-O-desosaminyl-6-O-methylerythronolide A 11,12-cyclic carbamate

### Example 11: 11-Amino-11-N-[4-[4-(3-pyridyl) imidazol-1-yl]butyl-11-deoxy-3-oxo-5-O-desosaminyl-6-O-methylerythronolide A 11,12-cyclic carbamate [Telithromycin] (Formula I)

10g of 2'-hydroxy -11-amino-11-N-[4-[4-(3-pyridyl) imidazol-1-yl] butyl]-11-deoxy-5-O-desosaminyl-6-O-methylerythronolide A 11,12-cyclic carbamate was dissolved in 200 ml dichloromethane and 15g of Dess Martin reagent was added in one lot. The mixture was stirred at ambient temperature for 30 min. Further, 260 ml of saturated sodium bicarbonate solution added and stirred the mixture for 30 min. Filtered off the solid precipitate and separated the organic layer. Washed the organic layer with water, dried over sodium sulfate and distilled off the solvent under vacuum to give solids. Further, it was stirred with 40ml of diisoproyl ether and filtered off and dried to give 9gm 11-Amino-11-N-[4-[4-(3 pyridyl) imidazol-1-yl]butyl-11-deoxy-3-oxo-5-O-desosaminyl-6-O-methylerythronolide A 11,12-cyclic carbamate [Telithromycin](I).

### Example 12: 11-Amino-11-N-[4-[4-(3-pyridyl) imidazol-1-yl]butyl-11-deoxy-3-oxo-5-O-desosaminyl-6-O-methylerythronolide A 11,12-cyclic carbamate [Telithromycin] (Formula I)

10g of 2'-hydroxy -11-amino-11-N-[4-[4-(3-pyridyl) imidazol-1-yl] butyl]-11-deoxy-5-O-desosaminyl-6-O-methylerythronolide A 11,12-cyclic carbamate was dissolved in 100 ml dichloromethane. Dimethyl sulfoxide (16.6 ml), Cyclohexyl dimethyl amino propyl carbodimide Hydro chloride (25.0g) and Pyridine HCl (12.1 gm) was added in one lot. The mixture was stirred at ambient temperature (20-30°C) for 6 hour. Further, 500 ml water was added and stirred the mixture for 30 min. The organic layer was separated and washed with water, dried over sodium sulfate and distilled off the solvent under vacuum to give 8.05 gm of 11-Amino-11-N-[4-[4-(3-pyridyl) imidazol-1-yl]butyl-11-deoxy-3-oxo-5-O-desosaminyl-6-O-methylerythronolide A 11,12-cyclic carbamate [Telithromycin](I).

### Example 13: 11-Amino-1-N-[4-[4-(3-pyridyl)imidazol-1-yl]butyl-11-deoxy-3-oxo-5-O-desosaminyl-6-O-methylerythronolide A 11,12-cyclic carbamate [Telithromycin] (Formula I)

N-Chloro succinimide (4.68 gm) was charged to the reaction vessel under nitrogen atmosphere and Dichloromethane (200 ml) was added slowly. The reaction masses was cooled to 0°C, Dimethyl sulfide (3.5 ml) was added slowly and continue the stirring at same temperature for half an hour. The reaction mass was cooled to -25°C and 2'-hydroxy -11-amino-11-N-[4-[4-(3-pyridyl)imidazol-1-yl] butyl]-11-deoxy-5-O-desosaminyl-6-O-methylerythronolide A 11,12-cyclic carbamate (10g) dissolved in 50 ml dichloromethane was added slowly. The mixture was stirred at -25°C temperature for 2 hour. At the same temperature Diisopropylethyl amine (0.6ml) was added and the reaction mixture was stirred for half an hour. Water (500 ml) was added and stirred the mixture for 30 min. The organic layer was separated and washed with water, dried over sodium sulfate and distilled off the solvent under vacuum to give 6.5 gm of 11-Amino-11-N-[4-[4-(3-pyridyl) imidazol-1-yl]butyl-11-deoxy-3-oxo-5-O-desosaminyl-6-O-methylerythronolide A 11,12-cyclic carbamate [Telithromycin] (Formula I).

### Example 14: Preparation of 2',4"-di-O-benzoyl-6-O-methylerythromycin A (IXa)

1250 ml of ethyl acetate was added to 250 gm Clarithromycin A. 264.65 g benzoic anhydride, 57.20g 4-dimethylamino pyridine and 67.60g tri ethyl amine was added to the reaction mixture at 25°C to 35°C. The reaction mixture was stirred for about 70 hours at ambient temperature After the completion of reaction, ethyl acetate was distilled out to obtain 2',4"-di-O-benzoyl-6-O-methylerythromycin A

### Example 15: Preparation of 10,11-anhydro-2',4"-di-O-benzoyl-12-O-imidazolylcarbonyl-6-O-methylerythromycin A (Xa)

1000 ml Dimethylformamide is added to 2',4"-di-O-benzoyl-6-O-methylerythromycin A at 25°C to35°C. 63.70 g DBU (1,8-Diazabicyclo[5.4.0]undec-7-ene) was added to the reaction mixture and stirred at ambient temperature. Further, 170 g 1,1-Carbonyl diimidazole was added to the reaction mass and it was stirred until completion of reaction at ambient temperature. The desired compound is isolated by addition of water

### Example 16: Preparation of 2',4"-di-O-benzoyl-11-amino-11-N-[4-[4-(3-pyridyl)imidazol-1-yl]butyl]-11-deoxy-6-O-methylerythromycin A 11,12-cyclic carbamate (XIa)

1000 ml dimethylformamide was added to 200 g 10,11-anhydro-2',4"-di-O-benzoyl-12-O-imidazolylcarbonyl-6-O-methylerythromycin A at 25°C to 35°C. 63 g of 4-[4-(3-pyridyl)imidazol-1-yl]butylamine and 29.50 g DBU was added to the reaction mixture and it was stirred at 25°C to 35°C until the completion of reaction, . It is further treated with cold water and obtained solid was treated with dichloromethane followed by extraction and removal of solvent to give 2',4"-di-O-benzoyl-11-amino-11-N-[4-[4-(3-pyridyl)imidazol-1-yl]butyl]-11-deoxy-6-O-methylerythromycin A 11,12-cyclic carbamate.

### Example 17: Preparation of 2'-O-benzoyl-11-amino-11-N-[4-[4-(3-pyridyl)imidazol-1-yl]butyl]-11-deoxy-5-O-desosaminyl-6-O-methylerythronolide A 11,12-cyclic carbamate (XIIa)

400 ml acetone was added to 200 g 2',4"-di-O-benzoyl-11-amino-11-N-[4-[4-(3-pyridyl)imidazol-1-yl]butyl]-11-deoxy-6-O-methylerythromycin A 11,12-cyclic carbamate to obtain clear solution at 25°C to 35°C. Dilute hydrochloric acid (400 ml) was added to the reaction mixture and it was stirred for 24 hours at ambient temperature. After the completion of the reaction, the reaction mixture was extracted with ethyl acetate and treated with Sodium hydroxide solution to give 2'-O-benzoyl-11-amino-11-N-[4-[4-(3-pyridyl)imidazol-1-yl]butyl]-11-deoxy-5-O-desosaminyl-6-O-methylerythronolide A 11,12-cyclic carbamate (XIIa)

### Example 18: Preparation of 2'-benzoyl-11-amino-11-N-[4-[4-(3-pyridyl)imidazol-1-yl]butyl]-11-deoxy-5-O-desosaminyl-6-O-methylerythronolide A 11,12-cyclic carbamate (XIIIa)

180 ml dichloromethane was added to 8.0 g N-chlorosuccinimide under nitrogen at room temperature cooled to 0°C. 7.2 ml dimethylsulfide was added slowly to the reaction mixture at 0°C under stirring. 20 g 2'-O-benzoyl-11-amino-11-N-[4-[4-(3-pyridyl)imidazol-1-yl]butyl]-11-deoxy-5-O-desosaminyl-6-O-methylerythronolide A 11,12-cyclic carbamate dissolved in 80ml dichloromethane was added drop wise to the reaction mixture at 0°C under stirring. Further it was cooled to about -20°C and solution of 16 ml Triethylamine in 20 ml dichloromethane is added to the reaction mixture and stirred for 30 minutes. After completion of the reaction, it is treated with saturated sodium bicarbonate solution and organic layer separated out. Desired product is obtained from by distillation of solvent from organic layer

### Example 19: Preparation of 11,12-dideoxy-3-de[(2,6-dideoxy-3-C-methyl-3-O-methyl-alpha-L-ribohexopyranosyl)oxy]-6-O-methyl-3-oxo-12,11-(oxycarbonyl[4-[4-(3-pyridinyl)-1H-imidazol-1-yl]butyl]imino)-erythromycin. (Telithromycin)

100 ml methanol was added to 10 g 2'-benzoyl-11-amino-11-N-[4-[4-(3-pyridyl)imidazol-1-yl]butyl]-11-deoxy-5-O-desosaminyl-6-O-methylerythronolide A 11,12-cyclic carbamate at 25°C to 30°C and the reaction mixture was heated to reflux for about 7 hours. After completion of the reaction, Methanol was distilled off under vacuum at 45°C. 100 ml dilute hydrochloric acid was added to the residue and the aqueous layer was extracted with ethyl acetate (3 x 40 ml) and sodium bicarbonate and organic layer separated out. The product is obtained by distillation of solvent from organic layer and recrystallized from Metyl tert- butyl ether (MTBE) and cyclohexanone to give Telithromycin.

## Claims

1. A process for the preparation of compound of formula (I) (Telithromycin) or its pharmaceutically acceptable salts 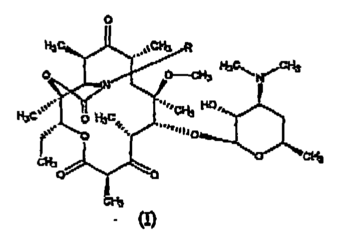 where, R is 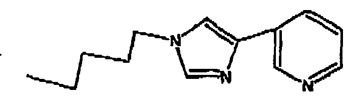 the process comprising the steps of
(a) reacting compound of formula (IX) 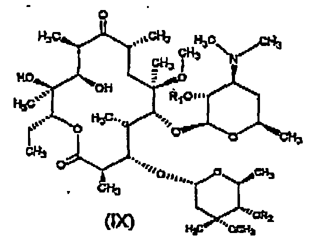 with carbonyldiimidazole in presence of a polar solvent and base to obtain the compound of formula (X) 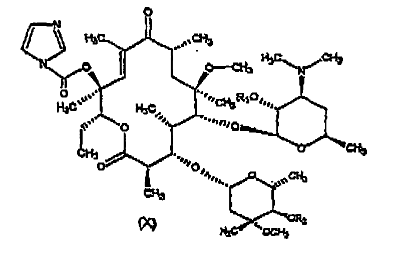 where R₁ and R₂ are same or different protecting groups represented by 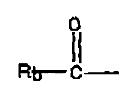 R_{b} is C₁ to C₁₀ alkyl group or aryl group, preferably R_{b} is C₁ - C₄ alkyl group, aryl represents substituted or unsubstituted phenyl group; more preferably R₁ and R₂ are same or different selected from acetyl, benzyl or benzoyl group
(b) condensing the compounds of formula (X) with R-NH₂ in a suitable polar solvent to obtain compounds of formula (XI) 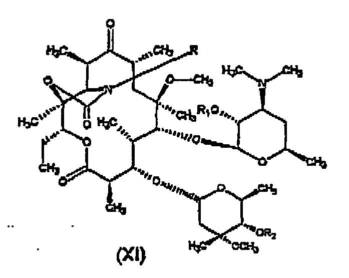 where R is as defined above and R₁ and R₂ are same or different protecting groups as described above;
(c) treating the obtained compound formula (XI) with an acid to give the compound of formula (XII) 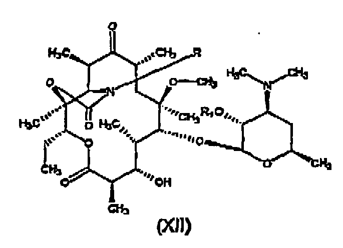
(d) oxidizing the resulting compounds of formula (XII) in presence of oxidizing agent to form compounds of formula (XIII) 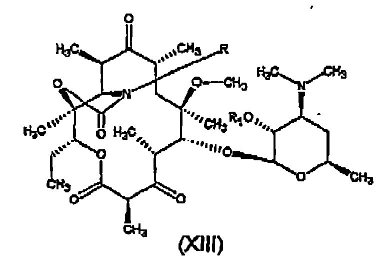
(e) removing the protecting group at 2' position of formula (XIII) by treating with an alcohol to give Telithromycin of Formula (I)

2. A process for the preparation of compounds of formula (I) or its pharmaceutically acceptable salts 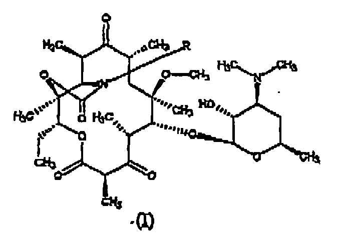 where, R is 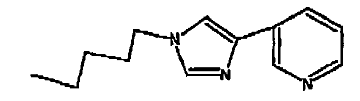 the process comprising the steps of
(a) reacting compound of formula (IX) 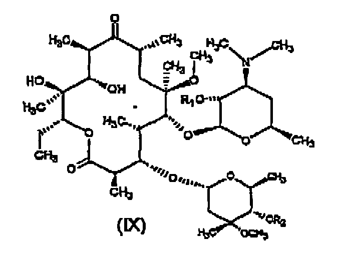 with carbonyldiimidazole in presence of a polar solvent and base to obtain the compound of formula (X), 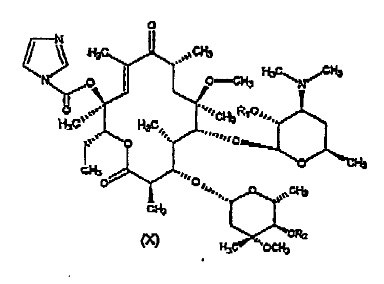 where R₁ and R₂ are same or different protecting groups represented by 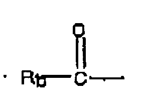 R_{b} is C₁ to C₁₀ alkyl group or aryl group, preferably R_{b} is C₁ - C₄ alkyl group, aryl represents substituted or unsubstituted phenyl group, more preferably R₁ and R₂ are same or different selected from acetyl, benzyl or benzoyl group;
(b) condensing the compounds of formula (X) with R-NH₂ in a suitable polar solvent to obtain compounds of formula (XI) 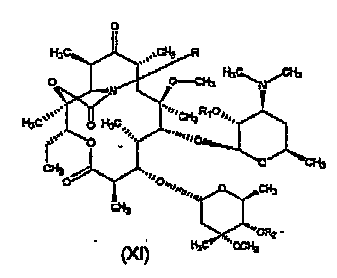 where R is as defined above and R₁ and R₂ are same or different protecting groups as described above;
(c) treating the obtained compound formula (XI) with an acid to give compound of formula (XII) 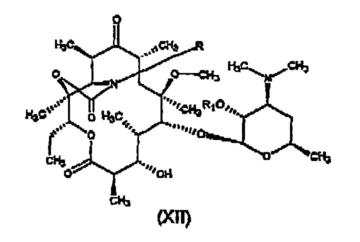
(f) treating compounds of formula (XII) with an alcohol to give compounds of formula (XIV) 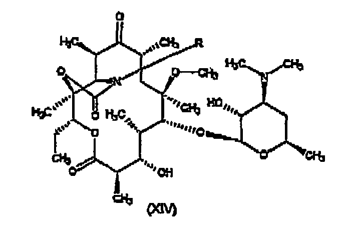
(g) oxidizing the resulting compounds of formula (XIV) of step (f) selectively in presence of oxidizing agent to obtain Telithromycin formula (I).

3. A process as claimed in claim 1, wherein said polar solvent in step (a) is selected from dimethylformamide, tetrahydrofuran, acetonitrile and mixtures thereof.

4. A process as claimed in claim 1, wherein said base in step (a) is selected from DBU, triethylamine, diisopropylethylamine.

5. A process as claimed in claim 1, wherein said polar solvent in step (b) is selected from group comprising of methanol, ethanol, isopropanol, n-propanol, n-butatlol, iso butyl alcohol, tert-butyl alcohol, methoxyethanol, ethoxyethanol, pentanol, neo-pentyl alcohol, tert-pentyl alcohol, cyclohexanol, ethylene glycol, propylene glycol, benzyl alcohol, phenol, glycerol, dimethylformamide (DMF), dimethylacetamide (DMAC), 1,3-dimethyl-3,4,5,6-tetrahydro-2(1H)-pyrimidinone (DMPU), 1,3-dimethyl-2-imidazolidinone (DMI), N-methylpyrrolidinone (NMP), formamide, N-methylacetamide, N-methylformamide, acetonitrile, dimethylsulfoxide, propionitrile, ethyl formate, methyl acetate, hexachloroacetone, HMPA, HMPT, acetone, ethyl methyl ketone, ethyl acetate, isopropyl acetate, t-butyl acetate, sulfolane, N,N-dimethylpropionamide, nitromethane, nitrobenzene, tetrahydrofuran (THF), dioxane, water, polyethers or mixtures thereof.

6. A process as claimed in claim 5, wherein said polar solvent is selected from dimethylformamide or acetonitrile.

7. A process as claimed in claim 1, wherein said step (b) is carried out in presence or absence of base selected from DBU, triethylamine, diisopropylethylamine

8. A process as claimed in claim 1, wherein said step (b) is carried out at a temperature 5° C to 120° C.

9. A process as claimed in claim 8, wherein the said step (b) is carried out preferably at a temperature 30°C to 60° C.

10. A process as claimed in claim 1, wherein the said acid in step (c) is selected from organic or inorganic acid.

11. A process as claimed in claim 10, wherein the said acid is selected from the group comprising of hydrochloric acid, hydrobromic acid, sulfuric acid, nitric acid, phosphoric acid, perchloric acid or hydrofluoric acid.

12. A process as claimed in claim 11, wherein the acid is preferably hydrochloric acid.

13. A process as claimed in claim 1, wherein step (c) is carried out in a solvent selected from water, polar organic solvents or mixtures thereof.

14. A process as claimed in claim 13, wherein said solvent is selected from water, alcohol or mixtures thereof.

15. A process as claimed in claim 14, wherein said solvent is selected from water, methanol, ethanol, isopropanol, n-propanol, tert-butanol, n-butanol or mixtures thereof

16. A process as claimed in claim 1, wherein said step (c) is carried out at a temperature 0°C to 70° C

17. A process as claimed in claim 16, where in step (c) is carried out at a temperature 20°C to 60° C

18. A process as claimed in claim 1, wherein oxidation said in step (d) is carried out using Corey- Kim oxidation method, Dess- Martin reagent, Pfitzner Moffat method or modifications thereof or with dimethyl sulfoxide in presence of oxalyl chloride or phosphorous pentoxide or p-Toluene sulfonyl chloride or acetic anhydride or N-chlorosuccinimide or by manganese or chromium or selenium reagents, tert-amine oxides or any said oxidant in presence or absence of phase transfer catalyst.

19. A process as claimed in claim 1, wherein alcohol said in step (e) is selected from group comprising of methanol, ethanol, n-propanol, iso propanol, tert-butanol, n-butanol or mixtures thereof.

20. A process as claimed in claim 19, wherein the said alcohol is preferably methanol.

21. A process as claimed in claim 1, wherein said step (e) is carried out in presence or absence of mineral acid selected from HCl, H₂SO₄

22. A process as claimed in claim 1, wherein said step (e) is carried out at a temperature of 0°C to 100°C

23. A process as claimed in claim 22, wherein step (e) is carried out preferably at a temperature of 20°C to 70°C.

24. A process as claimed in claim 2, wherein said alcohol in step (f) is selected from group comprising of methanol, ethanol, -n-propanol, iso propanol, tert-butanol, n-butanol or mixtures thereof.

25. A process as claimed in claim 24, wherein said alcohol is preferably methanol.

26. A process as claimed in claim 2, wherein said step (g) is carried out at a temperature of 0 to 70°C.

27. A process as claimed in claim 26, wherein the temperature is between 20 to 65°C.

28. A process as claimed in claim 2, wherein said oxidation in step (g) is carried out using Corey- Kim oxidation method, Dess- Martins reagent, Pfitzner moffat method or modifications thereof or with dimethyl sulfoxide in presence of oxalyl chloride or phosphorous pentoxide or p-Toluene sulfonyl chloride or acetic anhydride or N-chlorosucinimide.

29. A process as claimed in claim 2, wherein oxidation in step (g) is carried out by manganese or chromium or selenium reagents, tert-amine oxides or any above oxidant in presence of phase transfer catalyst.

30. The novel compounds
(i) 10,11-Anhydro-2',4"-di-O-benzoyl-12-O-imidazolylcarbonyl-6-O-methylerythromycin A of formula (Xa) 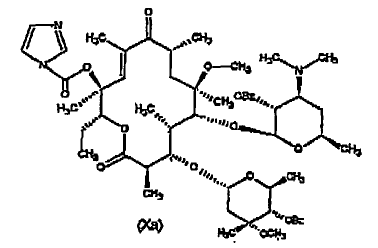
(ii) 2',4"-di-O-benzoyl-11-amino-11-N-[4-[4-(3-pyridyl)imidazol-11-yl]butyl]-11-deoxy-6-O-methylerythromycin A 11,12-cyclic carbamate of formula (XIa) 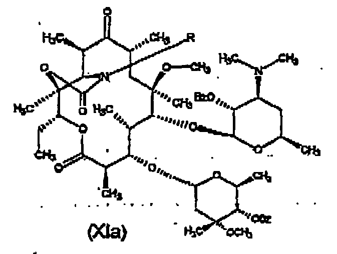
(iii) 2'-O-benzoyl-11-amino-11-N-[4-[4-(3-pyridyl)imidazol-1-yl]butyl]-11-deoxy-5-O-desosaminyl-6-O-methylerythronolide A 11,12-cyclic carbamate of formula (XIIa) 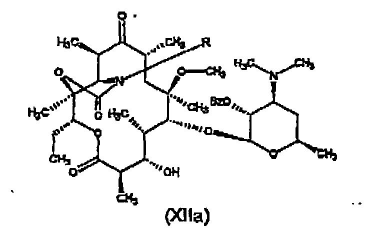
(iv) 2'-benzoyl-11-amino-11-N-[4-[4-(3-pyridyl)imidazol-1-yl]butyl-11-deoxy-5-O-desosaminyl-6-O-methylerythronolide A 11,12-cyclic carbamate of formula (XIIIa) 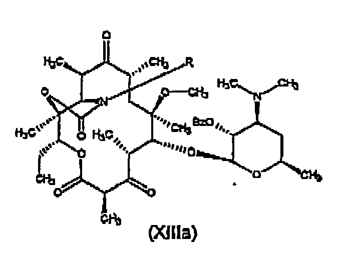
where R is

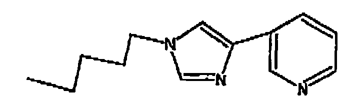

31. A process for the preparation of compound of formula (XIIIa) 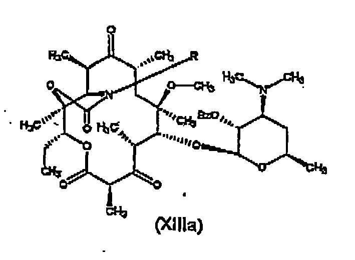 where, R is 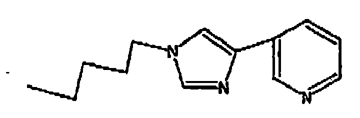 the process composing the steps of
(a') reacting 2',4"-di-O-benzoyl-6-O-methylerythromycin A compound of formula (IXa) 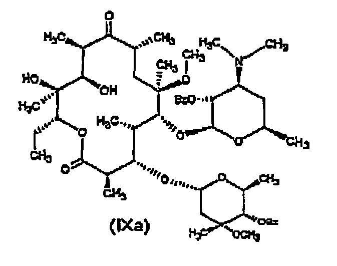 with carbonyldiimidazole in presence of a polar solvent and base to obtain 10,11-anhydro-2',4"-di-O-benzoyl-12-O-imidazolylcarbonyl-6-O-methylerythromycin A (Xa) 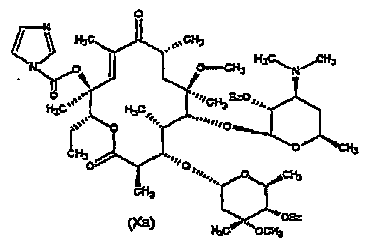
(b') condensing the compounds of formula (Xa) with R-NH₂ in a suitable polar solvent in presence of base to obtain 2',4"-di-O-benzoyl-11-amino-11-N-[4-[4-(3-pyridyl)imidazol-1-yl]butyl]-11-deoxy-6-O-methylerythromycin A 11,12-cyclic carbamate of formula (XIa) 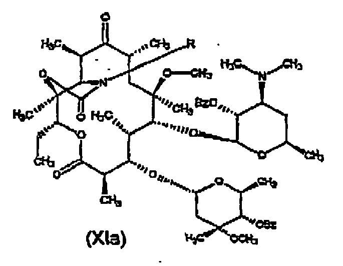 where R is as defined above
(c') treating the obtained compound formula (XIa) with an acid to give 2'-O-benzoyl-11-amino-11-N-[4-[4-(3-pyridyl)imidazol-1-yl]butyl]-11-deoxy-5-O-desosaminyl-6-O-methylerythronolide A 11,12-cyclic carbamate of formula (XIIa) 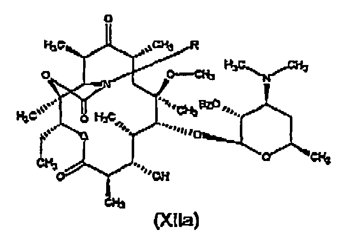
(d') oxidizing the resulting compounds of formula (XIIa) in presence of oxidizing agent to obtain the compound of formula (XIIIa)

32. A process as claimed in claim 31, wherein said polar solvent in step (a') is selected from dimehtylformamide, tetrahydrofuran, acetonitrile and mixtures thereof

33. A process as claimed in claim 31, wherein said base in step (a)' is selected from DBU, triethylamine, diisopropylethylamine.

34. A process as claimed in claim 31, wherein said polar solvent in step (b') is selected from group comprising of methanol, ethanol, isopropanol, n-propanol, n-butanol, iso butyl alcohol, tert-butyl alcohol, methoxyethanol, ethoxyethanol, pentanol, neo-pentyl alcohol, tert-pentyl alcohol, cyclohexanol, ethylene glycol, propylene glycol, benzyl alcohol, phenol, glycerol, dimethylformamide (DMF), dimethylacetamide (DMAC), 1,3-dimethyl-3,4,5,6-tetrahydro-2(1H)-pyrimidinone (DMPU), 1,3-dimethyl-2-imidazolidinone (DMI), N-methylpyrrolidinone (NMP), formamide, N-methylacetamide, N-methylformamide, acetonitrile, dimethylsulfoxide, propionitrile, ethyl formate, methyl acetate, hexachloroacetone, HMPA, HMPT, acetone, ethyl methyl ketone, ethyl acetate, isopropyl acetate, t-butyl acetate, sulfolane, N,N-dimethylpropianamide, nitromethane, nitrobenzene, tetrahydrofuran (THF), dioxane, water, polyethers or mixtures thereof.

35. A process as claimed in claim 31, wherein said polar solvent is selected from dimethylformamide or acetonitrile.

36. A process as claimed in claim 31, wherein base said in step (b') is selected from DBU, triethylamine, diisopropylethylamine.

37. A process as claimed in claim 31, wherein the said step (b') is carried out preferably at a temperature 30°C to 60° C.

38. A process as claimed in claim 31, wherein the said acid in step (c') is selected from organic or inorganic acid selected from the group comprising of hydrochloric acid, hydrobromic acid, sulfuric acid, nitric acid, phosphoric acid, perchloric acid or hydrofluoric acid.

39. A process as claimed in claim 31, wherein step (c') is carried out in a solvent selected from water, methanol, ethanol, isopropanol, n-propanol, tert-butanol, n-butanol or mixtures thereof.

40. A process as claimed in claim 31, where in step (c') is carried out at a temperature 20°C to 60°C

41. A process as claimed in claim 31, wherein oxidation said in step (d') is carried out using Corey- Kim oxidation method, Dess- Martin reagent, Pfitzner Moffat method or modifications thereof or with dimethyl sulfoxide in presence of oxalyl chloride or phosphorous pentoxide or p-Toluene sulfonyl chloride or acetic anhydride or N-chlorosuccinimide by manganese or chromium or selenium reagents, tert-amine oxides or any said oxidant in presence of phase transfer catalyst.

42. Use of compounds of formula (Xa), (XIa), (XIIa), (XIIIa) for the preparation of Telithromycin (I)

## Patentansprüche

1. Verfahren zur Herstellung einer Verbindung der Formel (I) (Telithromycin) oder deren pharmazeutisch annehmbaren Salzes 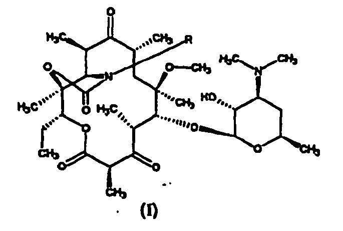 worin R 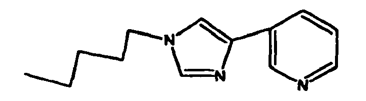 ist, das Verfahren die Schritte umfasst des
(a) Umsetzens der Verbindung der Formel (IX) 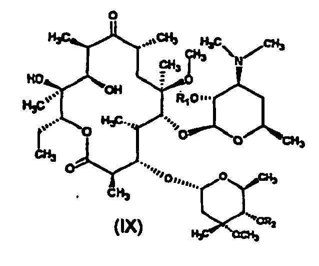 mit Carbonyldiimidazol in Gegenwart eines polaren Lösemittels und Base um die Verbindung der Formel (X) zu erhalten 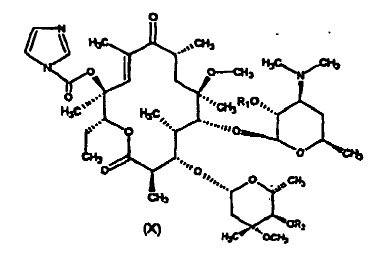 worin R₁ and R₂ gleiche oder unterschiedliche Schutzgruppen sind, dargestellt durch 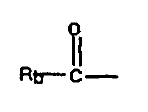 R_{b} ist C₁ bis C₁₀ Alkylgruppe oder Arylgruppe, bevorzugt ist R_{b} C₁ - C₄ Alkylgruppe, Aryl stellt substituierte oder unsubstituierte Phenylgruppe dar; stärker bevorzugt sind R₁ und R₂ gleich oder unterschiedlich ausgewählt aus Acetyl-, Benzyl- oder Benzoylgruppe
(b) Kondensierens der Verbindungen der Formel (X) mit R-NH₂ in einem geeigneten polaren Lösemittel um Verbindungen der Formel (XI) zu erhalten 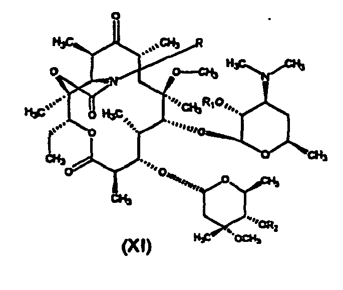 worin R wie oben definiert ist und R₁ and R₂ gleiche oder unterschiedliche Schutzgruppen wie oben beschrieben sind;
(c) Behandelns der erhaltenen Verbindung nach Formel (XI) mit einer Säure um die Verbindung der Formel (XII) zu ergeben 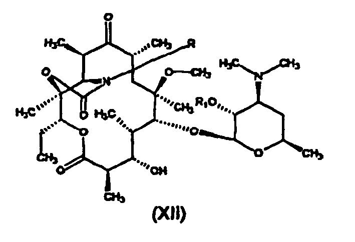
(d) Oxidierens der erhaltenen Verbindungen der Formel (XII) in Gegenwart eines Oxidationsmittels um Verbindungen der Formel (XIII) zu bilden 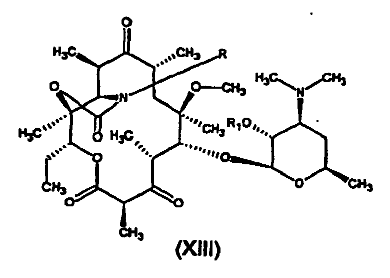
(e) Entfernens der Schutzgruppe an der 2'-Position von Formel (XIII) durch Behandeln mit einem Alkohol um Telithromycin der Formel (I) zu ergeben.

2. Verfahren zur Herstellung von Verbindungen der Formel (I) oder deren pharmazeutisch annehmbaren Salzen 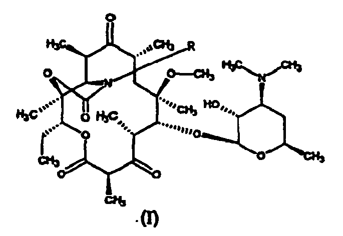 worin R 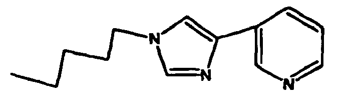 ist, das Verfahren die Schritte umfasst des
(a) Umsetzens der Verbindung der Formel (IX) 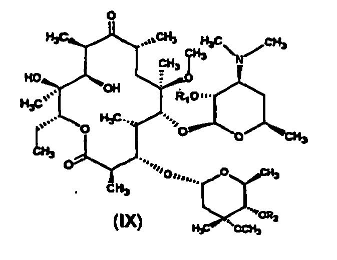 mit Carbonyldiimidazol in Gegenwart eines polaren Lösemittels und Base um die Verbindung der Formel (X) zu erhalten 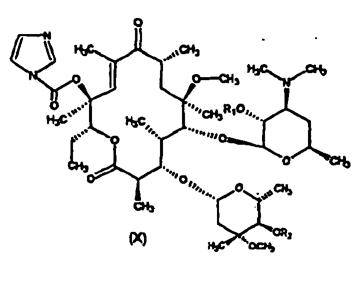 worin R₁ and R₂ gleiche oder unterschiedliche Schutzgruppen sind, dargestellt durch 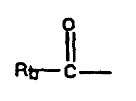 R_{b} ist C₁ bis C₁₀ Alkylgruppe oder Arylgruppe, bevorzugt ist R_{b} C₁ - C₄ Alkylgruppe, Aryl stellt substituierte oder unsubstituierte Phenylgruppe dar; stärker bevorzugt sind R₁ und R₂ gleich oder unterschiedlich ausgewählt aus Acetyl-, Benzyl- oder Benzoylgruppe
(b) Kondensierens der Verbindungen der Formel (X) mit R-NH₂ in einem geeigneten polaren Lösemittel um Verbindungen der Formel (XI) zu ergeben 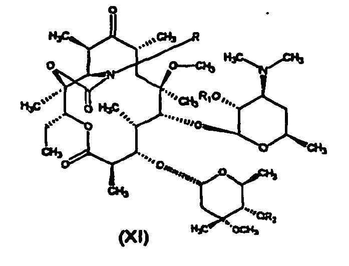 worin R wie oben definiert ist und R₁ and R₂ gleiche oder unterschiedliche Schutzgruppen wie oben beschrieben sind;
(c) Behandelns der erhaltenen Verbindung nach Formel (XI) mit einer Säure um die Verbindung der Formel (XII) zu ergeben 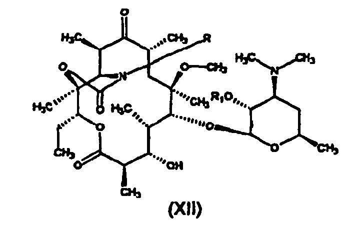
(f) Behandelns der Verbindungen der Formel (XII) mit einem Alkohol um Verbindungen der Formel (XIV) zu ergeben 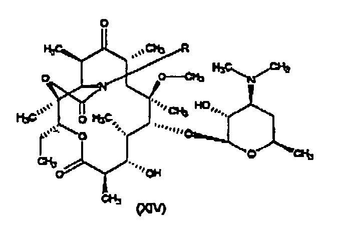
(g) Oxidierens der erhaltenen Verbindungen der Formel (XIV) aus Schritt (f) selektiv in Gegenwart eines Oxidationsmittels um Telithromycin der Formel (I) zu erhalten.

3. Verfahren wie in Anspruch 1 beansprucht, worin das genannte polare Lösemittel im Schritt (a) aus Dimethylformamid, Tetrahydrofuran, Acetonitril und deren Mischungen ausgewählt ist.

4. Verfahren wie in Anspruch 1 beansprucht, worin genannte Base in Schritt (a) aus DBU, Triethylamin, Diisopropylethylamin ausgewählt ist.

5. Verfahren wie in Anspruch 1 beansprucht, worin das genannte polare Lösemittel im Schritt (b) aus der Gruppe bestehend aus Methanol, Ethanol, Isopropanol, n-Propanol, n-Butanol, iso-Butylalkohol, tert.-Butylalkohol, Methoxyethanol, Ethoxyethanol, Pentanol, neo-Pentylalkohol, tert.-Pentylalkohol, Cyclohexanol, Ethylenglykol, Propylenglykol, Benzylalkohol, Phenol, Glycerin, Dimethylformamid (DMF), Dimethylacetamid (DMAC), 1,3-Dimethyl-3,4,5,6-tetrahydro-2(1H)-pyrimidinon (DMPU), 1,3-Dimethyl-2-imidazolidinon (DMI), N-Methylpyrrolidinon (NMP), Formamid, N-Methylacetamid, N-Methylformamid, Acetonitril, Dimethylsulfoxid, Propionitril, Ethylformiat, Methylacetat, Hexachloraceton, HMPA, HMPT, Aceton, Ethylmethylketon, Ethylacetat, Isopropylacetat, t-Butylacetat, Sulfolan, N,N-Dimethylpropionamid, Nitromethan, Nitrobenzol, Tetrahydrofuran (THF), Dioxan, Wasser, Polyether oder deren Mischungen ausgewählt ist.

6. Verfahren wie in Anspruch 5 beansprucht, worin das genannte polare Lösemittel aus Dimethylformamid oder Acetonitril ausgewählt ist.

7. Verfahren wie in Anspruch 1 beansprucht, worin der genannte Schritt (b) in Gegenwart oder Abwesenheit von Base ausgewählt aus DBU, Triethylamin, Diisopropylethylamin ausgeführt wird.

8. Verfahren wie in Anspruch 1 beansprucht, worin der genannte Schritt (b) bei einer Temperatur von 5 °C bis 120 °C ausgeführt wird.

9. Verfahren wie in Anspruch 8 beansprucht, worin der genannte Schritt (b) vorzugsweise bei einer Temperatur von 30 °C bis 60 °C ausgeführt wird.

10. Verfahren wie in Anspruch 1 beansprucht, worin die genannte Säure in Schritt (c) aus organischen oder anorganischen Säuren ausgewählt ist.

11. Verfahren wie in Anspruch 10 beansprucht, worin die genannte Säure aus der Gruppe bestehend aus Salzsäure, Bromwasserstoffsäure, Schwefelsäure, Salpetersäure, Phosphorsäure, Perchlorsäure oder Fluorwasserstoffsäure ausgewählt ist.

12. Verfahren wie in Anspruch 11 beansprucht, worin die Säure vorzugsweise Salzsäure ist.

13. Verfahren wie in Anspruch 1 beansprucht, worin Schritt (c) in einem Lösemittel ausgewählt aus Wasser, polaren organischen Lösemitteln oder deren Mischungen durchgeführt wird.

14. Verfahren wie in Anspruch 13 beansprucht, worin das genannte Lösemittel aus Wasser, Alkohol oder deren Mischungen ausgewählt ist.

15. Verfahren wie in Anspruch 14 beansprucht, worin das genannte Lösemittel aus Wasser, Methanol, Ethanol, Isopropanol, n-Propanol, tert.-Butanol, n-Butanol oder deren Mischungen ausgewählt ist.

16. Verfahren wie in Anspruch 1 beansprucht, worin der genannte Schritt (c) bei einer Temperatur von 0 °C bis 70 °C ausgeführt wird.

17. Verfahren wie in Anspruch 16 beansprucht, worin Schritt (c) bei einer Temperatur von 20 °C bis 60 °C ausgeführt wird.

18. Verfahren wie in Anspruch 1 beansprucht, worin die in Schritt (d) genannte Oxidation unter Verwendung der Corey-Kim-Oxidationsmethode, des Dess-Martin-Reagenz, der Pfitzner-Moffatt-Methode oder deren Modifikationen oder mit Dimethylsulfoxid in Gegenwart von Oxalylchlorid oder Phosphorpentoxid oder p-Toluolsulfonylchlorid oder Acetanhydrid oder N-Chlorsuccinimid oder durch Mangan- oder Chrom- oder Selen-Reagenzien, tert.-Aminoxide oder jedes der genannten Oxidationsmittel in Gegenwart oder Abwesenheit von Phasentransferkatalysatoren ausgeführt wird.

19. Verfahren wie in Anspruch 1 beansprucht, worin der in Schritt (e) genannte Alkohol aus der Gruppe bestehend aus Methanol, Ethanol, n-Propanol, Isopropanol, tert.-Butanol, n-Butanol oder deren Mischungen ausgewählt ist.

20. Verfahren wie in Anspruch 19 beansprucht, worin der genannte Alkohol vorzugsweise Methanol ist.

21. Verfahren wie in Anspruch 1 beansprucht, worin der genannte in Schritt (e) in Gegenwart oder Abwesenheit einer Mineralsäure ausgewählt aus HCl, H₂SO₄ durchgeführt wird.

22. Verfahren wie in Anspruch 1 beansprucht, worin der genannte Schritt (e) bei einer Temperatur von 0 °C bis 100 °C ausgeführt wird.

23. Verfahren wie in Anspruch 22 beansprucht, worin Schritt (e) vorzugsweise bei einer Temperatur von 20 °C bis 70 °C ausgeführt wird.

24. Verfahren wie in Anspruch 2 beansprucht, worin der in Schritt (f) genannte Alkohol ausgewählt ist aus der Gruppe enthaltend Methanol, Ethanol, n-Propanol, Isopropanol, tert.-Butanol, n-Butanol oder deren Mischungen.

25. Verfahren wie in Anspruch 24 beansprucht, worin der genannte Alkohol vorzugsweise Methanol ist.

26. Verfahren wie in Anspruch 2 beansprucht, worin der genannte Schritt (g) bei einer Temperatur von 0 °C bis 70 °C ausgeführt wird.

27. Verfahren wie in Anspruch 26 beansprucht, worin die Temperatur 20 °C bis 65 °C ist.

28. Verfahren wie in Anspruch 2 beansprucht, worin die genannte Oxidation in Schritt (g) unter Verwendung der Corey-Kim-Oxidationsmethode, des Dess-Martin-Reagenz, der Pfitzner-Moffatt-Methode oder deren Modifikationen oder mit Dimethylsulfoxid in Gegenwart von Oxalylchlorid oder Phosphorpentoxid oder p-Toluolsulfonylchlorid oder Acetanhydrid oder N-Chlorsuccinimid ausgeführt wird.

29. Verfahren wie in Anspruch 2 beansprucht, worin die Oxidation in Schritt (g) durch Mangan- oder Chrom- oder Selen-Reagenzien, tert.-Aminoxide oder jedes obige Oxidationsmittel in Gegenwart von Phasentransferkatalysatoren ausgeführt wird.

30. Die neuen Verbindungen
(i) 10,11-Anhydro-2',4"-di-O-benzoyl-12-O-imidazolylcarbonyl-6-O-methylerythromycin A der Formel (Xa) 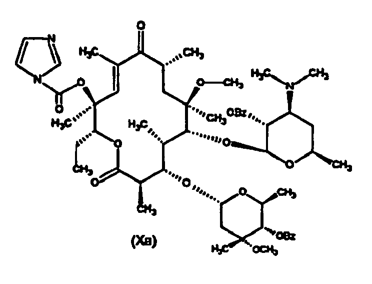
(ii) 2',4"-Di-O-benzoyl-11-amino-11-N-[4-[4-(3-pyridyl)imidazol-1-yl]butyl]-11-deoxy-6-O-methylerythromycin A 11,12-cyclisches Carbamat der Formel (XIa) 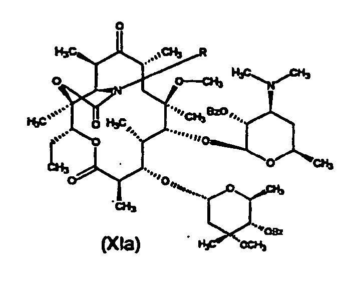
(iii) 2'-O-Benzoyl-11-amino-11-N-[4-[4-(3-pyridyl)imidazol-1-yl]butyl]-11-deoxy-5-O-desosaminyl-6-O-methylerythronolid A 11,12-cyclisches Carbamat der Formel (XIIa) 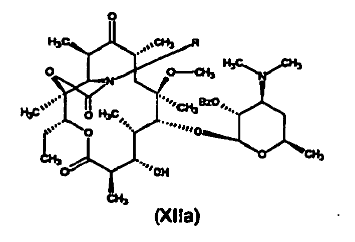
(iv) 2'-Benzoyl-11-amino-11-N-[4-[4-(3-pyridyl)imidazol-1-yl]butyl]-11-deoxy-5-O-desosaminyl-6-O-methylerythronolid A 11,12-cyclisches Carbamat der Formel (XIIIa) 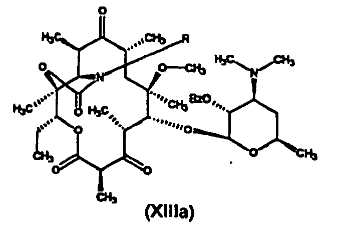
worin R 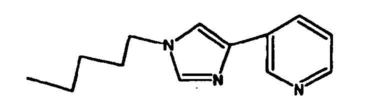 ist.

31. Verfahren zur Herstellung der Verbindung der Formel (XIIIa) 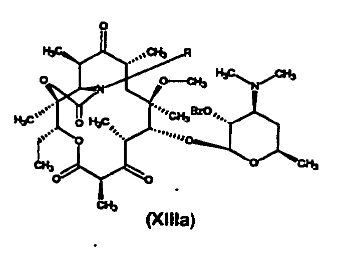 worin R 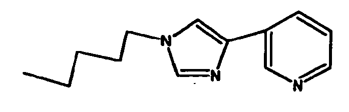 ist, das Verfahren die Schritte umfasst des
(a') Umsetzens von 2',4"-Di-O-benzoyl-6-O-methylerythromycin A der Verbindung der Formel (IXa) 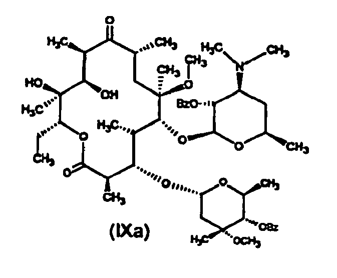 mit Carbonyldiimidazol in Gegenwart eines polaren Lösemittels und Base um 10,11-Anhydro-2',4"-di-O-benzoyl-12-O-imidazolylcarbonyl-6-O-methylerythromycin A (Xa) zu erhalten 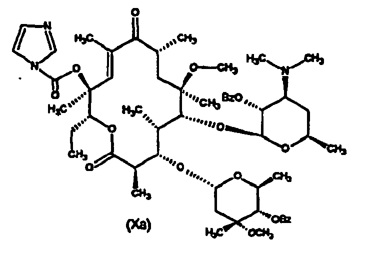
(b') Kondensierens der Verbindungen der Formel (Xa) mit R-NH₂ in einem geeigneten polaren Lösemittel in Gegenwart von Base um 2',4"-Di-O-benzoyl-11-amino-11-N-[4-[4-(3-pyridyl)imidazol-1-yl]butyl]-11-deoxy-6-O-methylerythromycin A 11,12-cyclisches Carbamat der Formel (XIa) zu erhalten 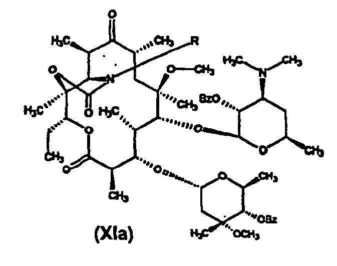 worin R wie oben definiert ist
(c') Behandelns der erhaltenen Verbindung der Formel (XIa) mit einer Säure um 2'-O-Benzoyl-11-amino-11-N-[4-[4-(3-pyridyl)imidazol-1-yl]butyl]-11-deoxy-5-O-desosaminyl-6-O-methylerythronolide A 11,12-cyclisches Carbamat der Formel (XIIa) zu ergeben 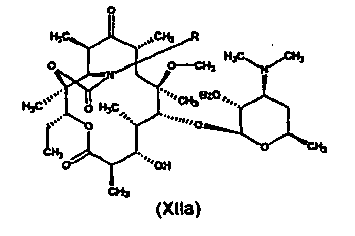
(d') Oxidierens der erhaltenden Verbindungen der Formel (XIIa) in Gegenwart eines Oxidationsmittels um die Verbindung der Formel (XIIIa) zu ergeben.

32. Verfahren wie in Anspruch 31 beansprucht, worin das genannte polare Lösemittel in Schritt (a') aus Dimethylformamid, Tetrahydrofuran, Acetonitril und deren Mischungen ausgewählt ist.

33. Verfahren wie in Anspruch 31 beansprucht, worin die genannte Base in Schritt (a') aus DBU, Triethylamin, Diisopropylethylamin ausgewählt ist.

34. Verfahren wie in Anspruch 31 beansprucht, worin das genannte polare Lösemittel in Schritt (b') aus der Gruppe bestehend aus Methanol, Ethanol, Isopropanol, n-Propanol, n-Butanol, Isobutylalkohol, tert.-Butylalkohol, Methoxyethanol, Ethoxyethanol, Pentanol, neo-Pentylalkohol, tert.-Pentylalkohol, Cyclohexanol, Ethylenglycol, Propylenglycol, Benzylalkohol, Phenol, Glycerin, Dimethylformamid (DMF), Dimethylacetamid (DMAC), 1,3-Dimethyl-3,4,5,6-tetrahydro-2(1H)-pyrimidinon (DMPU), 1,3-Dimethyl-2-imidazolidinon (DMI), N-Methylpyrrolidinon (NMP), Formamid, N-Methylacetamid, N-Methylformamid, Acetonitril, Dimethylsulfoxid, Propionitril, Ethylformiat, Methylacetat, Hexachloraceton, HMPA, HMPT, Aceton, Ethylmethylketon, Ethylacetat, Isopropylacetat, t-Butylacetat, Sulfolan, N,N-Dimethylpropionamid, Nitromethan, Nitrobenzol, Tetrahydrofuran (THF), Dioxan, Wasser, Polyether oder deren Mischungen ausgewählt ist.

35. Verfahren wie in Anspruch 31 beansprucht, worin das genannte polare Lösemittel aus Dimethylformamid oder Acetonitril ausgewählt ist.

36. Verfahren wie in Anspruch 31 beansprucht, worin die genannte Base in Schritt (b') aus DBU, Triethylamin, Diisopropylethylamin ausgewählt ist.

37. Verfahren wie in Anspruch 31 beansprucht, worin der genannte Schritt (b') vorzugsweise bei einer Temperatur von 30 °C bis 60 °C ausgeführt wird.

38. Verfahren wie in Anspruch 31 beansprucht, worin die genannte Säure in Schritt (c') ausgewählt ist aus organischer oder anorganischer Säure, ausgewählt aus der Gruppe enthaltend Salzsäure, Bromwasserstoffsäure, Schwefelsäure, Salpetersäure, Phosphorsäure, Perchlorsäure oder Fluorwasserstoffsäure.

39. Verfahren wie in Anspruch 31 beansprucht, worin der Schritt (c') in einem Lösemittel ausgewählt aus Wasser, Methanol, Ethanol, Isopropanol, n-Propanol, tert.-Butanol, n-Butanol oder deren Mischungen ausgeführt wird.

40. Verfahren wie in Anspruch 31 beansprucht, worin der Schritt (c') bei einer Temperatur von 20 °C bis 60 °C ausgeführt wird.

41. Verfahren wie in Anspruch 31 beansprucht, worin die in Schritt (d') genannte Oxidation unter Verwendung der Corey-Kim-Oxidationsmethode, des Dess-Martin-Reagenz, der Pfitzner-Moffatt-Methode oder deren Modifikationen oder mit Dimethylsulfoxid in Gegenwart von Oxalylchlorid oder Phosphorpentoxid oder p-Toluolsulfonylchlorid oder Acetanhydrid oder N-Chlorsuccinimid durch Mangan- oder Chrom- oder Selen-Reagenzien, tert.-Aminoxide oder jedes der genannten Oxidationsmittel in Gegenwart von Phasentransferkatalysatoren ausgeführt wird.

42. Verwendung der Verbindungen der Formeln (Xa), (XIa), (XIIa),(XIIIa) für die Herstellung von Telithromycin (I).

## Revendications

1. Un procédé pour la préparation du composé de formule (I) (Télithromycine) ou de ses sels pharmaceutiquement acceptables 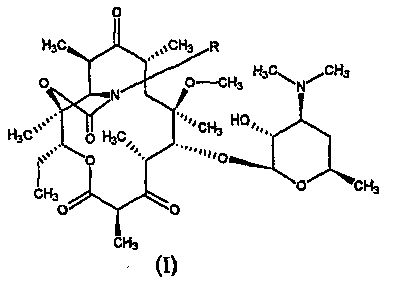 dans laquelle R est : 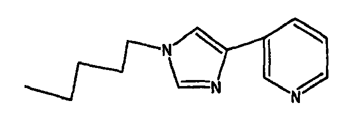 le procédé comportant les étapes suivantes consistant à
(a) faire réagir un composé de formule (IX) 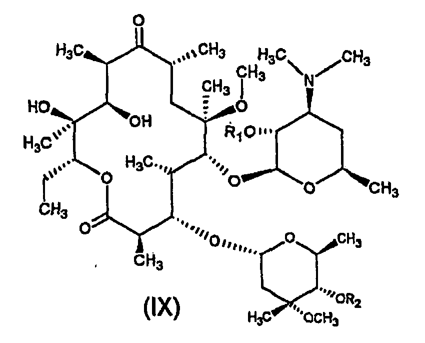 avec du carbonyldiimidazole en présence d'un solvant polaire et d'une base pour obtenir le composé de formule (X) 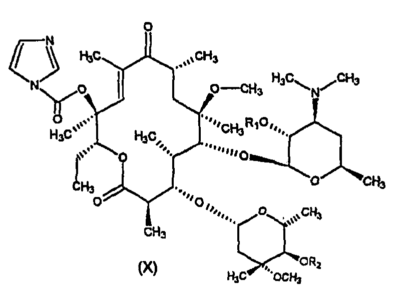 dans laquelle R₁ et R₂ sont des groupes protecteurs identiques ou différents représentés par 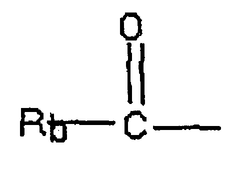 R_{b} est un groupe alkyle C₁ à C₁₀ ou un groupe aryle, de préférence un groupe alkyle C₁ - C₄, le groupe aryle représente un groupe phényle substitué ou non substitué ; R₁ et R₂ sont de préférence les mêmes ou différents choisis parmi les groupes acétyle, benzyle ou benzoyle,
(b) faire condenser les composés de formule (X) avec R-NH₂ dans un solvant polaire approprié pour obtenir des composés de formule (XI) 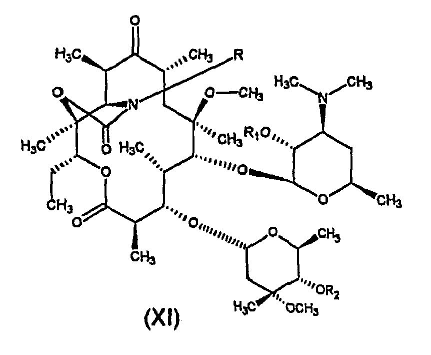 dans laquelle R est défini comme ci-dessus et R₁ et R₂ sont des groupes protecteurs identiques ou différents comme décrits ci-dessus ;
(c) traiter le composé obtenu de formule (XI) avec un acide pour donner le composé de formule (XII) 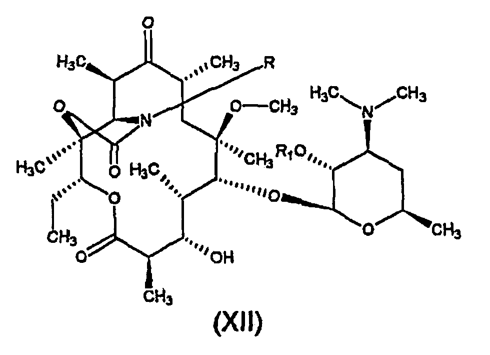
(d) oxyder les composés résultants de formule (XII) en présence d'un agent oxydant pour obtenir des composés de formule (XIII) 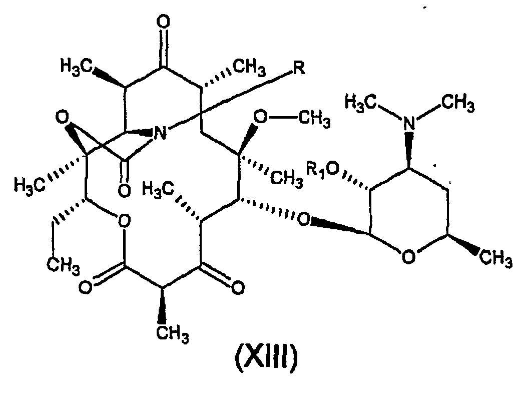
(e) enlever le groupe protecteur en position 2' dans la formule (XIII) en traitant les composés avec un alcool pour obtenir la Télithromycine selon la formule (I).

2. Un procédé pour la préparation du composé de formule (I) ou de ses sels pharmaceutiquement acceptables 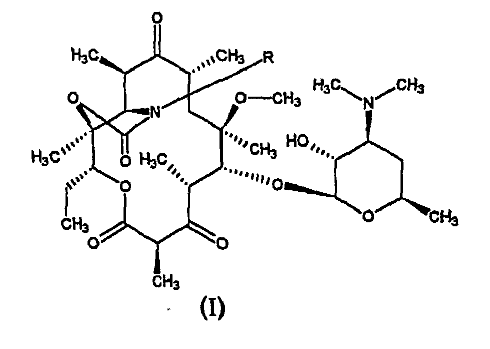 dans laquelle R est 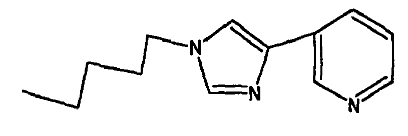 le procédé comportant les étapes suivantes consistant à
(a) faire réagir un composé de formule (IX) 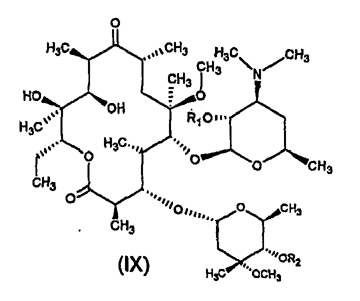 avec du carbonyldiimidazole en présence d'un solvant polaire et d'une base pour obtenir le composé de formule (X), 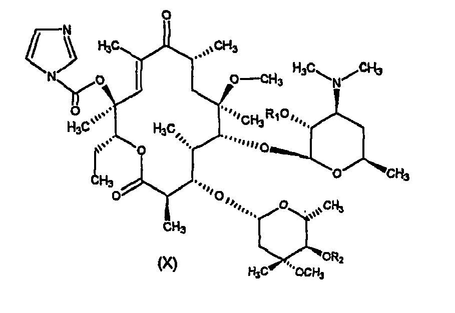 dans laquelle R₁ et R₂ sont des groupes protecteurs identiques ou différents représentés par 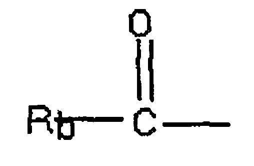 R_{b} est un groupe alkyle C₁ à C₁₀ ou un groupe aryle, de préférence un groupe alkyle C₁ - C₄, le groupe aryle représente un groupe phényle substitué ou non substitué ; R₁ et R₂ sont de préférence les mêmes ou différents choisis parmi les groupes acétyle, benzyle ou benzoyle;
(b) faire condenser les composés de formule (X) avec R-NH₂ dans un solvant polaire approprié pour obtenir des composés de formule (XI) 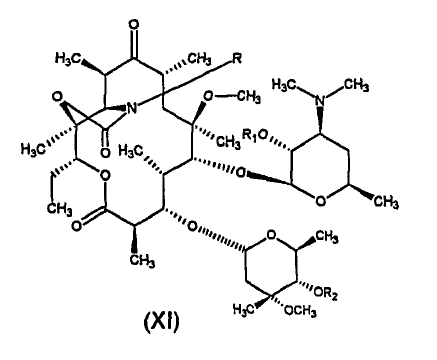 dans laquelle R est défini comme ci-dessus et R₁ et R₂ sont des groupes protecteurs identiques ou différents comme décrits ci-dessus ;
(c) traiter le composé obtenu de formule (XI) avec un acide pour donner le composé de formule (XII) 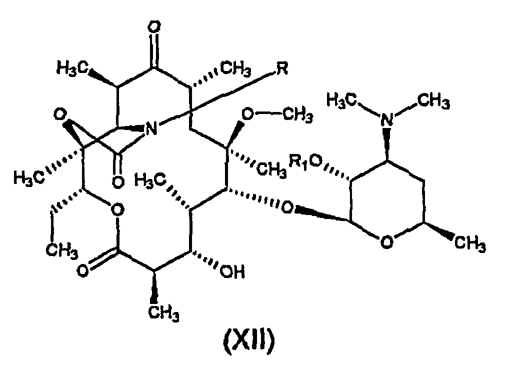
(f) traiter le composé de formule (XII) avec un alcool pour donner le composé de formule (XIV) 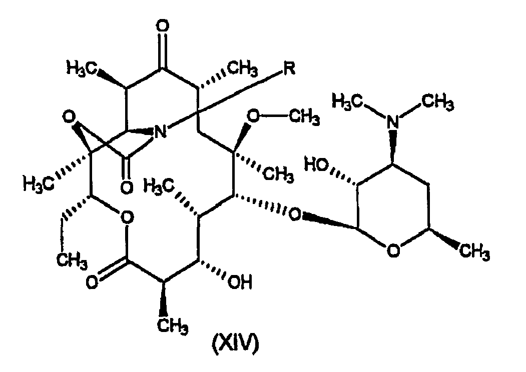
(g) oxyder le composé de formule (XIV) résultant de l'étape (f) sélectivement en présence d'un agent oxydant pour obtenir la formule de la Télithromycine (I).

3. Un procédé selon la revendication 1, dans lequel ledit solvant polaire dans l'étape (a) est choisi parmi la diméthylformamide, le tétrahydrofurane, l'acétonitrile et leurs mélanges.

4. Un procédé selon la revendication 1, dans lequel ladite base dans l'étape (a) est choisie parmi le DBU, la tri-éthylamine, la diisopropyléthylamine.

5. Un procédé selon la revendication 1, dans lequel ledit solvant polaire dans l'étape (b) est choisi parmi le groupe suivant : méthanol, éthanol, isopropanol, n-propanol, n-butanol, alcool iso butylique, alcool tert-butylique, méthoxyéthanol, éthoxyéthanol, pentanol, alcool néopentylique, alcool tert-pentylique, cyclohexanol, éthylène-glycol, propylène glycol, alcool benzylique, phénol, glycérol, diméthylformamide (DMF), diméthylacétamide (DMAC), 1,3-diméthyl-3,4,5,6-tétrahydro-2(1H)-pyrimidinone (DMPU), 1,3-diméthyl-2-imidazolidinone (DMI), N-méthylpyrrolidinone (NMP), formamide, N-méthylacétamide, N-méthylformamide, acétonitrile, diméthylsulfoxyde, propionitrile, formate d'éthyle, acétate de méthyle, hexachloroacétone, HMPA, HMPT, acétone, éthyl méthyl cétone, acétate d'éthyle, acétate d'isopropyle, acétate t-butylique, sulfolane, N,N-diméthylpropionamide, nitrométhane, nitrobenzène, tétrahydrofurane (THF), dioxane, eau, polyéthers ou leurs mélanges.

6. Un procédé selon la revendication 5, dans lequel ledit solvant polaire est choisi entre la diméthylformamide ou l'acétonitrile.

7. Un procédé selon la revendication 1, dans lequel ladite étape (b) est effectuée en la présence ou l'absence d'une base choisie parmi le DBU, la triéthylamine, la diisopropyléthylamine.

8. Un procédé selon la revendication 1, dans lequel ladite étape (b) est effectuée à une température entre 5° C et 120° C.

9. Un procédé selon la revendication 8, dans lequel ladite étape (b) est effectuée de préférence à une température entre 30° C et 60° C.

10. Un procédé selon la revendication 1, dans lequel ledit acide dans l'étape (c) est choisi parmi un acide organique ou non organique.

11. Un procédé selon la revendication 10, dans lequel ledit acide est choisi parmi le groupe suivant : acide chlorhydrique, acide bromhydrique, acide sulfurique, acide nitrique, acide phosphorique, acide perchlorique ou acide fluorhydrique.

12. Un procédé selon la revendication 11, dans lequel l'acide est de préférence de l'acide chlorhydrique.

13. Un procédé selon la revendication 1, dans lequel l'étape (c) est effectuée dans un solvant choisi parmi l'eau, les solvants organiques polaires ou des mélanges de ceux-ci.

14. Un procédé selon la revendication 13, dans lequel ledit solvant est choisi parmi l'eau, un alcool ou des mélanges de ceux-ci.

15. Un procédé selon la revendication 14, dans lequel ledit solvant est choisi parmi l'eau, le méthanol, l'éthanol, l'isopropanol, le n-propanol, le tert-butanol, le n-butanol ou des mélanges de ceux-ci.

16. Un procédé selon la revendication 1, dans lequel ladite étape (c) est effectuée à une température entre 0° C et 70° C

17. Un procédé selon la revendication 16, dans lequel dans l'étape (c) est effectuée à une température entre 20° C et 60° C.

18. Un procédé selon la revendication 1, dans lequel l'oxydation dans ladite étape (d) est effectuée en utilisant la méthode d'oxydation Corey-Kim, le réactif Dess-Martin, la méthode Pfitzner Moffat ou des modifications de ceux-ci ou avec du sulfoxyde diméthylique en présence de chlorure d'oxalyle ou de pentoxyde phosphoreux ou de chlorure p-Toluène sulfonyle ou d'anhydride acétique ou de N-chlorosuccinimide ou par des réactifs de manganèse ou de chrome ou de sélénium, des oxydes de tert-amine ou de tout dit oxydant en présence ou absence d'un catalyseur de transfert de phase.

19. Un procédé selon la revendication 1, dans lequel l'alcool dans ladite l'étape (e) est choisi parmi le groupe suivant : méthanol, éthanol, n-propanol, iso-propanol, tert-butanol, n butanol ou des mélanges de ceux-ci.

20. Un procédé selon la revendication 19, dans lequel ledit alcool est de préférence du méthanol.

21. Un procédé selon la revendication 1, dans lequel ladite étape (e) est effectuée en présence ou absence d'un acide minéral choisi parmi : HCl, H₂SO₄.

22. Un procédé selon la revendication 1, dans lequel ladite étape (e) est effectuée à une température entre 0° C et 100° C.

23. Un procédé selon la revendication 22, dans lequel l'étape (e) est effectuée de préférence à une température entre 20° C et 70° C.

24. Un procédé selon la revendication 2, dans lequel ledit alcool dans l'étape (f) est choisi parmi le groupe suivant : méthanol, éthanol, n-propanol, iso-propanol, tert-butanol, n-butanol ou des mélanges de ceux-ci.

25. Un procédé selon la revendication 24, dans lequel ledit alcool est de préférence du méthanol.

26. Un procédé selon la revendication 2, dans lequel ladite étape (g) est effectuée à une température entre 0° C et 70° C.

27. Un procédé selon la revendication 26, dans lequel la température est entre 20° C et 65° C.

28. Un procédé selon la revendication 2, dans lequel ladite oxydation dans l'étape (g) est effectuée en utilisant la méthode d'oxydation Corey-Kim, le réactif de Dess-Martin, la méthode Pfitzner Moffat ou des modifications de ceux-ci ou avec du sulfoxyde diméthylique en présence de chlorure d'oxalyle ou de pentoxyde phosphoreux ou de chlorure p-Toluène de sulfonyle ou d'anhydride acétique ou de N-chlorosucinimide.

29. Un procédé selon la revendication 2, dans lequel l'oxydation dans l'étape (g) est effectuée par des réactifs de manganèse ou de chrome ou de sélénium, des oxydes tert-amine ou de tout oxydant précédent en présence d'un catalyseur de transfert de phase.

30. Les nouveaux composés
(i) 10,11-Anhydro-2',4"-di-O-benzoyl-12-O-imidazolylcarbonyl-6-O-méthylérythromycine A de formule (Xa) 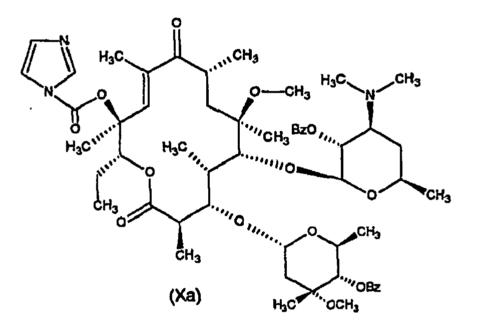
(ii) 11,12-carbamate cyclique de 2',4"-di-O-benzoyle-11-amino-11-N-[4-[4-(3-pyridyl)imidazol-1-yl]butyl]-11-deoxy-6-O-méthylérythromycine A de formule (XIa) 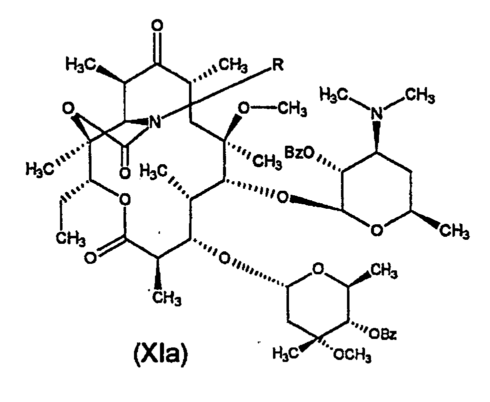
(iii) 11,12-carbamate cyclique de 2'-O-benzoyle-11-amino-11-N-[4-[4-(3-pyridyl)imidazol-1-yl]butyl)]-11-deoxy-5-O-desosaminyle-6-O-méthylérythronolide A de formule (XIIa) 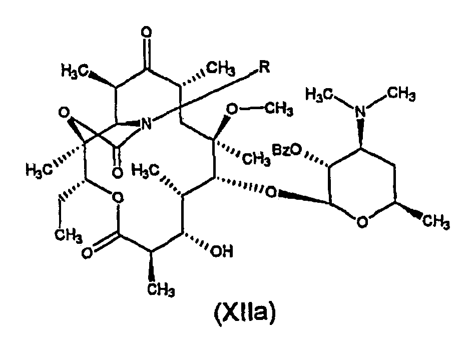
(iv) 11,12-carbamate cyclique de 2'-benzoyle-11-amino-11-N-[4-[4-(3-pyridyl)imidazol-1-yl]butyl]-11-deoxy-5-O-desosaminyl-6-O-méthylérythronolide A de formule (XIIIa) 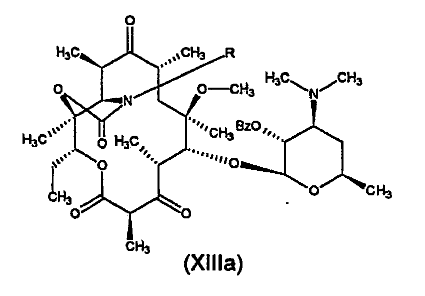
dans laquelle R est

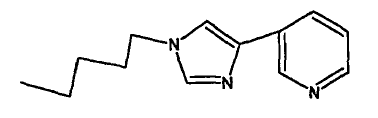

31. Un procédé pour la préparation d'un composé de formule (XIIIa) 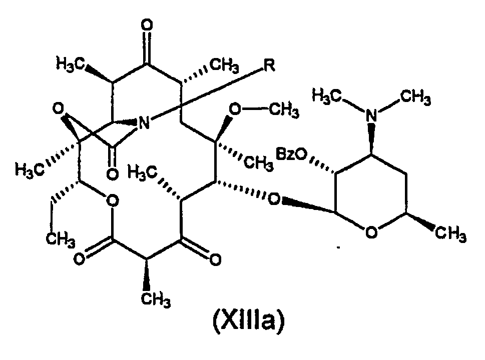 dans laquelle R est 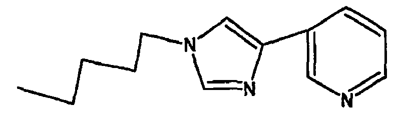 le procédé comportant les étapes consistant à :
(a') faire réagir un composé 2',4"-di-O-benzoyle-6-O-méthylérythromycine A de formule (IXa) 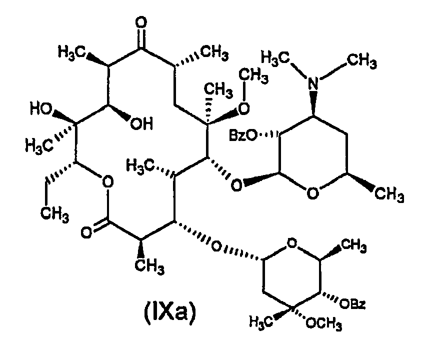
avec du carbonyldiimidazole en présence d'un solvant polaire et d'une base pour obtenir du 10,11-anhydro-2',4"-di-O-benzoyl-12-O-imidazolylcarbonyl-6-O-méthylérythromycine A (Xa) 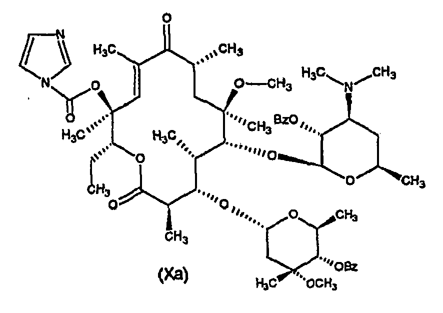
(b') faire condenser les composés de formule (Xa) avec R-NH₂ dans un solvant polaire approprié en présence d'une base pour obtenir du 11, 12-carbamate cyclique de 2',4"-di-O-benzoyle-11-amino-11-N-[4-[4-(3-pyridyl)imidazol-1-yl]butyl]-11-deoxy-6-O-méthylérythromycine A de formule (XIa) 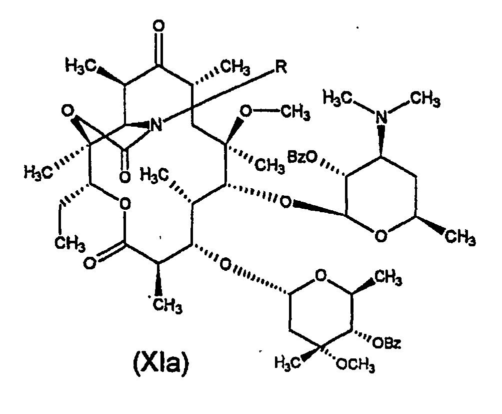 dans laquelle R est défini comme ci-dessus
(c') traiter le composant de formule (XIa) avec un acide pour donner du 11,12-carbamate cyclique de 2'-O-benzoyl-11-amino-11-N-[4-[4-(3-pyridyl)imidazol-1-yl]butyl]-11-deoxy-5-O-desosaminyl-6-O-méthylérythronolide A de formule (XIIa) 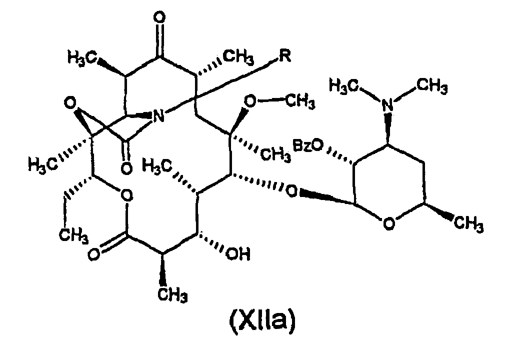
(d') oxyder les composés résultants de formule (XIIa) en présence d'un agent oxydant pour obtenir le composé de formule (XIIIa).

32. Un procédé selon la revendication 31, dans lequel ledit dissolvant polaire dans l'étape (a') est choisi parmi la diméthylformamide, le tétrahydrofurane, l'acétonitrile et leurs mélanges.

33. Un procédé selon la revendication 31, dans lequel ladite base dans l'étape (a') est choisie parmi le DBU, la triéthylamine, la diisopropyléthylamine.

34. Un procédé selon la revendication 31, dans lequel ledit solvant polaire dans l'étape (b') est choisi parmi le groupe suivant : méthanol, éthanol, isopropanol, n-propanol, n-butanol, alcool iso butylique, alcool tert-butylique, méthoxyéthanol, éthoxyéthanol, pentanol, alcool néopentylique, alcool tert-pentylique, cyclohexanol, éthylène-glycol, propylène glycol, alcool benzylique, phénol, glycérol, diméthylformamide (DMF), diméthylacétamide (DMAC), 1,3-diméthyl-3,4,5,6-tétrahydro-2(1H)-pyrimidinone (DMPU), 1,3-diméthyl-2-imidazolidinone (DMI), N-méthylpyrrolidinone (NMP), formamide, N-méthylacétamide, N-méthylformamide, acétonitrile, diméthylsulfoxyde, propionitrile, formate d'éthyle, acétate de méthyle, hexachloroacétone, HMPA, HMPT, acétone, éthyl méthyl cétone, acétate d'éthyle, acétate d'isopropyle, acétate t-butylique, sulfolane, N,N-diméthylpropionamide, nitrométhane, nitrobenzène, tétrahydrofurane (THF), dioxane, eau, polyéthers ou leurs mélanges.

35. Un procédé selon la revendication 31, dans lequel ledit solvant polaire est choisi entre la diméthylformamide ou l'acétonitrile.

36. Un procédé selon la revendication 31, dans lequel la base dans ladite étape (b') est choisie parmi le DBU, la triéthylamine, la diisopropyléthylamine.

37. Un procédé selon la revendication 31, dans lequel ladite étape (b') est effectuée de préférence à une température entre 30° C et 60° C.

38. Un procédé selon la revendication 31, dans lequel ledit acide dans l'étape (c') est choisi parmi un acide organique ou non organique choisi parmi le groupe suivant : acide chlorhydrique, acide bromhydrique, acide sulfurique, acide nitrique, acide phosphorique, acide perchlorique ou acide fluorhydrique.

39. Un procédé selon la revendication 31, dans lequel l'étape (c') est effectuée dans un solvant choisi parmi l'eau, le méthanol, l'éthanol, l'isopropanol, le n-propanol, le tert-butanol, le n-butanol ou des mélanges de ceux-ci.

40. Un procédé selon la revendication 31, dans lequel l'étape (c') est effectuée à une température entre 20° C et 60° C.

41. Un procédé selon la revendication 31, dans lequel l'oxydation dans l'étape (d') est effectuée en utilisant la méthode d'oxydation Corey-Kim, le réactif Dess-Martin, la méthode Pfitzner Moffat ou des modifications de ceux-ci ou avec du sulfoxyde de diméthyle en présence de chlorure d'oxalyle ou de pentoxyde phosphoreux ou de chlorure p-Toluène de sulfonyle ou d'anhydride acétique ou de N-chlorosuccinimide par des réactifs de manganèse ou de chrome ou de sélénium, des oxydes de tert-amine ou n'importe quel de ces oxydants en présence d'un catalyseur de transfert de phase.

42. Utilisation des composés de formules (Xa), (XIa), (XIIa), (XIIIa) pour la préparation de la Télithromycine (I).
